# EUROPEAN PATENT SPECIFICATION

(11) **EP 3 596 464 B1**
(45) Date of publication and mention of the grant of the patent: **11.08.2021**
(21) Application number: 18718697.8
(22) Date of filing: 14.03.2018
(51) Int. Cl.: G01N 33/53, C07K 16/18, G01N 33/58, G01N 33/60, C07K 16/42

(54) **TARGET DETECTION USING A MONOVALENT ANTIBODY**
ZIELDETEKTION UNTER VERWENDUNG EINES MONOVALENTEN ANTIKÖRPERS
DÉTECTION DE CIBLE À L'AIDE D'UN ANTICORPS MONOVALENT

(30) Priority: 14.03.2017 EP 17160720
(43) Date of publication of application: 22.01.2020
(73) Proprietor: NanoTag Biotechnologies GmbH, 37079 Göttingen (DE)
(72) Inventor: FREY, Steffen, 37075 Göttingen (DE); OPAZO, Felipe, 37083 Göttingen (DE); GÖTZKE, Hansjörg, 30171 Hannover (DE)
(74) Representative: Schiweck Weinzierl Koch Patentanwälte Partnerschaft mbB
(86) International application number: PCT/EP2018/056375
(87) International publication number: WO 2018/167141

(56) References cited:
- EP-A2- 2 259 067
- WO-A1-01/44301
- WO-A1-2007/047450
- US-A1- 2015 307 627
- MITO MARI ET AL: "Simultaneous multicolor detection of RNA and proteins using super-resolution microscopy", METHODS, ACADEMIC PRESS, US, vol. 98, 10 November 2015 (2015-11-10), pages 158-165, XP029471287, ISSN: 1046-2023, DOI: 10.1016/J.YMETH.2015.11.007
- TINO PLEINER ET AL: "A toolbox of anti-mouse and anti-rabbit IgG secondary nanobodies", THE JOURNAL OF CELL BIOLOGY : JCB, vol. 217, no. 3, 20 December 2017 (2017-12-20), pages 1143-1154, XP055466375, US ISSN: 0021-9525, DOI: 10.1083/jcb.201709115
- PLEINER TINO ET AL: "Nanobodies: site-specific labeling for super-resolution imaging, rapid epitope-mapping and native protein complex isolation", ELIFE, ELIFE SCIENCES PUBLICATIONS LTD, GB, vol. 4, 3 December 2015 (2015-12-03), pages e11349-1, XP009504731, ISSN: 2050-084X, DOI: 10.7554/ELIFE.11349
- Anonymous: "Primary and secondary antibodies", Wikipedia, 18 July 2016 (2016-07-18), XP055717879, Retrieved from the Internet: URL:https://en.wikipedia.org/w/index.php?t itle=Primary_and_secondary_antibodies&oldi d=730424125 [retrieved on 2020-07-24]

## Description

### FIELD OF THE INVENTION

The invention relates to methods of detecting a target antigen by optical detection, comprising contacting a first antibody with at least one camelid single domain antibody as defined in the claims that specifically binds said first antibody, wherein the camelid single domain antibody has one or more fluorescent label(s) attached to it. The invention also relates to complexes formed by the methods of the invention, as well as uses of the camelid single domain antibody according to the methods of the invention.

### BACKGROUND OF THE INVENTION

Immunofluorescence typically requires the detection of the protein of interest by a specific antibody. Typically, a primary antibody obtained from one specific animal species, e.g. rabbit, is used to bind the target antigen. A secondary antibody that recognizes specifically the primary antibody, to which a label is attached, is used to detect the primary antibody. This secondary antibody is typically fluorescently labeled and it derived from another species than the primary antibody.

The labeling with fluorescent molecules of the secondary antibody is typically performed with non-directed chemistry (i.e. random). As a result, the number of fluorescent molecules present in every single secondary antibody is not precisely known. Typically, it follows an average distribution between 0.5 to ∼ 3.5 fluorophores per secondary antibody, which will vary from distributor-to distributor and batch-to-batch, which makes imaging not always reproducible. The real meaning behind an average distribution of 0.5 to ∼3.5 fluorophores per secondary antibody means that some secondary antibodies have no fluorophore (meaning that the target will not be detected) and some have up to 5 or more fluorophores (which will result in a major overestimation of the detected target amounts).

The direct labeling of the primary antibody with fluorescent molecules is typically performed with non-directed chemistry (i.e. random). The direct coupling of fluorophores to primary antibodies is not often performed due to the risk of inactivating the binding capability of the antibody. This risk is especially high on monoclonal antibodies since adding a fluorophore on the paratope (epitope binding region on the antibody) will completely impair the antibody binding function of all molecules. Polyclonal antibodies might not be completely inactivated the coupling since there is a chance that some antibody molecules are not affected by the chemistry applied.

Another drawback of classical immunofluorescence approaches is that complexes of standard primary and secondary antibodies tend to form clusters or simply aggregates. Furthermore, the fluorophores are displaced from the desired target molecule between 15-25 nm. As current microscopes can have a resolution power of ~5 nm, the use of primary and secondary antibodies result in an error of 10-20 nm in all three dimensions from were the investigated target really is.

Time consuming and error prone experimental protocols are further limitation of classical immunofluorescence methods. Current protocols typically apply the primary antibody for a particular time, typically ranging from 0.5 to 24 h, and then wash off the excess of primary antibody that did not find any target. After washing, the secondary antibody is incubated for a particular time, typically ranging from 0.5 to 18 h, and then washed several times, typically for 10 to 30 min, to make sure no free fluorescently labeled secondary antibodies are left in the preparation. Here, especially washing steps are prone to errors which can easily result in a strong background signal that hampers the experiment.

Mari Mito et al., Methods 98 (2016) 158-165 discloses simultaneous multicolor detection of RNA and proteins using super-resolution microscopy.

Based on the above, there is a need in the art for improved means and methods for immunofluorescence.

Accordingly, the technical problem of the present invention is to comply with this need. This problem is solved by the embodiments of the independent claims.

### SUMMARY OF THE INVENTION

The present invention relates to a method of detecting a target antigen by optical detection, comprising:
contacting a first antibody with a target antigen that said first antibody specifically binds, or with a sample suspected to comprise said target antigen
contacting the first antibody with at least one second antibody that specifically binds the first antibody, wherein the second antibody is a camelid single domain antibody having one or more fluorescent label(s) attached to it,
wherein the camelid single domain antibody is selected from the group consisting of:
   (a) a camelid single domain antibody that specifically binds guinea pig IgG and that is not cross-reactive to the following antibody species: mouse IgG, rat IgG, chicken IgY, rabbit IgG, human IgG, human IgM, goat IgG, donkey IgG, pig IgG, horse IgG, and cattle IgG;
   (b) a camelid single domain antibody that specifically binds chicken IgY and that is not cross-reactive to the following antibody species: guinea pig IgG, mouse IgG, rat IgG, human IgG, human IgM, goat IgG, donkey IgG, pig IgG, horse IgG, and cattle IgG;
   (c) a camelid single domain antibody that specifically binds mouse or rat IgG and that is not cross-reactive to the following antibody species: guinea pig IgG, chicken IgY, rabbit IgG, human IgG, human IgM, goat IgG, donkey IgG, pig IgG, horse IgG, and cattle IgG;
   (d) a camelid single domain antibody that specifically binds human IgM and that is not cross-reactive to the following antibody species: guinea pig IgG, mouse IgG, rat IgG, chicken IgY, rabbit IgG, goat IgG, donkey IgG, pig IgG, horse IgG, and cattle IgG;
   (e) a camelid single domain antibody that specifically binds rabbit IgG and that is not cross-reactive to the following antibody species: guinea pig IgG, mouse IgG, rat IgG, chicken IgY, human IgG, human IgM, goat IgG, donkey IgG, pig IgG, horse IgG, and cattle IgG;
   (f) a camelid single domain antibody that specifically binds goat IgG and that is not cross-reactive to the following antibody species: guinea pig IgG, mouse IgG, rat IgG, human IgG, human IgM, chicken IgY, rabbit IgG, donkey IgG, pig IgG, horse IgG, and cattle IgG;
   (g) a camelid single domain antibody that specifically binds rat IgG and that is not cross-reactive to the following antibody species: guinea pig IgG, mouse IgG, chicken IgY, rabbit IgG, human IgG, human IgM, goat IgG, donkey IgG, pig IgG, horse IgG, and cattle IgG;
   (h) a camelid single domain antibody that specifically binds mouse IgG and that is not cross-reactive to the following antibody species: guinea pig IgG, rat IgG, chicken IgY, rabbit IgG, human IgG, human IgM, goat IgG, donkey IgG, pig IgG, horse IgG, and cattle IgG;
   (i) a camelid single domain antibody that specifically binds human IgG and that is not cross-reactive to the following antibody species: guinea pig IgG, mouse IgG, rat IgG, chicken IgY, rabbit IgG, human IgM, goat IgG, donkey IgG, pig IgG, horse IgG, and cattle IgG;
   (j) a camelid single domain antibody that specifically binds donkey IgG and that is not cross-reactive to the following antibody species: guinea pig IgG, mouse IgG, rat IgG, chicken IgY, rabbit IgG, human IgG, human IgM, goat IgG, pig IgG, horse IgG, and cattle IgG; and
   (k) a camelid single domain antibody that specifically binds sheep IgG and that is not cross-reactive to the following antibody species: guinea pig IgG, mouse IgG, rat IgG, chicken IgY, rabbit IgG, human IgG, human IgM, goat IgG, donkey IgG, pig IgG, horse IgG, and cattle IgG.

The present invention also relates to a complex comprising
a first antibody; and
at least one second antibody, wherein the at least one second antibody is bound to the first antibody via the antigen binding site of the second antibody, wherein the second antibody is a camelid single domain antibody having one or more fluorescent label(s) attached to it, wherein the camelid single domain antibody is selected from the group consisting of:
   (a) a camelid single domain antibody that specifically binds guinea pig IgG and that is not cross-reactive to the following antibody species: mouse IgG, rat IgG, chicken IgY, rabbit IgG, human IgG, human IgM, goat IgG, donkey IgG, pig IgG, horse IgG, and cattle IgG;
   (b) a camelid single domain antibody that specifically binds chicken IgY and that is not cross-reactive to the following antibody species: guinea pig IgG, mouse IgG, rat IgG, human IgG, human IgM, goat IgG, donkey IgG, pig IgG, horse IgG, and cattle IgG;
   (c) a camelid single domain antibody that specifically binds mouse or rat IgG and that is not cross-reactive to the following antibody species: guinea pig IgG, chicken IgY, rabbit IgG, human IgG, human IgM, goat IgG, donkey IgG, pig IgG, horse IgG, and cattle IgG;
   (d) a camelid single domain antibody that specifically binds human IgM and that is not cross-reactive to the following antibody species: guinea pig IgG, mouse IgG, rat IgG, chicken IgY, rabbit IgG, goat IgG, donkey IgG, pig IgG, horse IgG, and cattle IgG;
   (e) a camelid single domain antibody that specifically binds rabbit IgG and that is not cross-reactive to the following antibody species: guinea pig IgG, mouse IgG, rat IgG, chicken IgY, human IgG, human IgM, goat IgG, donkey IgG, pig IgG, horse IgG, and cattle IgG;
   (f) a camelid single domain antibody that specifically binds goat IgG and that is not cross-reactive to the following antibody species: guinea pig IgG, mouse IgG, rat IgG, human IgG, human IgM, chicken IgY, rabbit IgG, donkey IgG, pig IgG, horse IgG, and cattle IgG;
   (g) a camelid single domain antibody that specifically binds rat IgG and that is not cross-reactive to the following antibody species: guinea pig IgG, mouse IgG, chicken IgY, rabbit IgG, human IgG, human IgM, goat IgG, donkey IgG, pig IgG, horse IgG, and cattle IgG;
   (h) a camelid single domain antibody that specifically binds mouse IgG and that is not cross-reactive to the following antibody species: guinea pig IgG, rat IgG, chicken IgY, rabbit IgG, human IgG, human IgM, goat IgG, donkey IgG, pig IgG, horse IgG, and cattle IgG;
   (i) a camelid single domain antibody that specifically binds human IgG and that is not cross-reactive to the following antibody species: guinea pig IgG, mouse IgG, rat IgG, chicken IgY, rabbit IgG, human IgM, goat IgG, donkey IgG, pig IgG, horse IgG, and cattle IgG;
   (j) a camelid single domain antibody that specifically binds donkey IgG and that is not cross-reactive to the following antibody species: guinea pig IgG, mouse IgG, rat IgG, chicken IgY, rabbit IgG, human IgG, human IgM, goat IgG, pig IgG, horse IgG, and cattle IgG;
   (k) a camelid single domain antibody that specifically binds sheep IgG and that is not cross-reactive to the following antibody species: guinea pig IgG, mouse IgG, rat IgG, chicken IgY, rabbit IgG, human IgG, human IgM, goat IgG, donkey IgG, pig IgG, horse IgG, and cattle IgG.

The present invention also relates to a use of a camelid single domain antibody having one or more fluorescent label(s) attached to it as a secondary antibody for detecting a target antigen by optical detection,
wherein the camelid single domain antibody is selected from the group consisting of:
(a) a camelid single domain antibody that specifically binds guinea pig IgG and that is not cross-reactive to the following antibody species: mouse IgG, rat IgG, chicken IgY, rabbit IgG, human IgG, human IgM, goat IgG, donkey IgG, pig IgG, horse IgG, and cattle IgG;
(b) a camelid single domain antibody that specifically binds chicken IgY and that is not cross-reactive to the following antibody species: guinea pig IgG, mouse IgG, rat IgG, human IgG, human IgM, goat IgG, donkey IgG, pig IgG, horse IgG, and cattle IgG;
(c) a camelid single domain antibody that specifically binds mouse or rat IgG and that is not cross-reactive to the following antibody species: guinea pig IgG, chicken IgY, rabbit IgG, human IgG, human IgM, goat IgG, donkey IgG, pig IgG, horse IgG, and cattle IgG;
(d) a camelid single domain antibody that specifically binds human IgM and that is not cross-reactive to the following antibody species: guinea pig IgG, mouse IgG, rat IgG, chicken IgY, rabbit IgG, goat IgG, donkey IgG, pig IgG, horse IgG, and cattle IgG;
(e) a camelid single domain antibody that specifically binds rabbit IgG and that is not cross-reactive to the following antibody species: guinea pig IgG, mouse IgG, rat IgG, chicken IgY, human IgG, human IgM, goat IgG, donkey IgG, pig IgG, horse IgG, and cattle IgG;
(f) a camelid single domain antibody that specifically binds goat IgG and that is not cross-reactive to the following antibody species: guinea pig IgG, mouse IgG, rat IgG, human IgG, human IgM, chicken IgY, rabbit IgG, donkey IgG, pig IgG, horse IgG, and cattle IgG;
(g) a camelid single domain antibody that specifically binds rat IgG and that is not cross-reactive to the following antibody species: guinea pig IgG, mouse IgG, chicken IgY, rabbit IgG, human IgG, human IgM, goat IgG, donkey IgG, pig IgG, horse IgG, and cattle IgG;
(h) a camelid single domain antibody that specifically binds mouse IgG and that is not cross-reactive to the following antibody species: guinea pig IgG, rat IgG, chicken IgY, rabbit IgG, human IgG, human IgM, goat IgG, donkey IgG, pig IgG, horse IgG, and cattle IgG;
(i) a camelid single domain antibody that specifically binds human IgG and that is not cross-reactive to the following antibody species: guinea pig IgG, mouse IgG, rat IgG, chicken IgY, rabbit IgG, human IgM, goat IgG, donkey IgG, pig IgG, horse IgG, and cattle IgG;
(j) a camelid single domain antibody that specifically binds donkey IgG and that is not cross-reactive to the following antibody species: guinea pig IgG, mouse IgG, rat IgG, chicken IgY, rabbit IgG, human IgG, human IgM, goat IgG, pig IgG, horse IgG, and cattle IgG; and
(k) a camelid single domain antibody that specifically binds sheep IgG and that is not cross-reactive to the following antibody species: guinea pig IgG, mouse IgG, rat IgG, chicken IgY, rabbit IgG, human IgG, human IgM, goat IgG, donkey IgG, pig IgG, horse IgG, and cattle IgG.

### BRIEF DESCRIPTION OF THE DRAWINGS

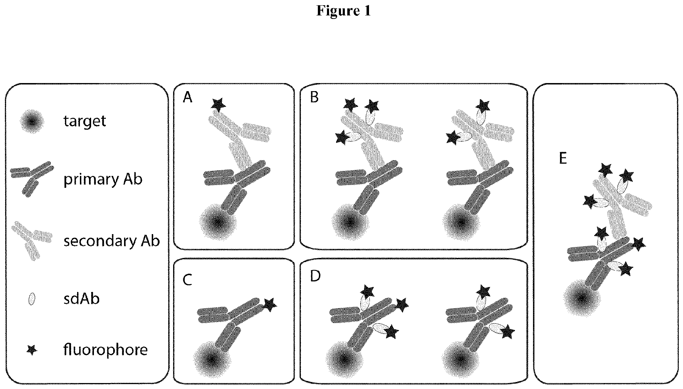

**Figure 1** Comparison of conventional immunofluorescence with sdAb-boosted immunofluorescence (scheme). **(A):** Conventional indirect immunofluorescence: A primary antibody directly binding the target protein is recognized by a fluorescently labeled secondary antibody. The number of fluorescent labels on each secondary antibody is not fixed but is generally a broad distribution with average values between 0.5 to ∼ 3.5 fluorophores per antibody molecule. The resulting signal intensity is thus not directly proportional to the number of targets. **(B):** Two examples of immunofluorescence boosted by fluorescently labeled sdAb. First, the signal of the conventional approach **(A)** is boosted by binding two labeled monovalent antibodies to the secondary antibody. Since exactly two monovalent antibodies (each site-specifically labeled with a defined number of fluorophores) can bind per secondary antibody, the additional label is added in a stoichiometric manner. This label can be used for enhancing an already existing signal of the secondary antibody (e.g. if a labels with similar spectral properties are used for the secondary antibody and the sdAb). Second, the fluorescently labeled sdAb can be used as the only (stoichiometrically defined) signal or used as an alternative signal that is better suited for quantitative detection of the target (e.g. if a different or distinguishing labels are used for secondary antibody and the sdAb). **(C):** Conventional direct immunofluorescence detection. This approach is not used extensively due to the high risk of hampering the target binding ability of the primary antibody by the coupling procedure. **(D):** Two alternatives uses of fluorescently labeled sdAb in direct immunofluorescence staining. First, sdAbs can be used to enhance the signal of the labeled primary antibody. Second, the primary antibody can be labeled fluorescently using sdAbs without any risk of hampering its binding to the target molecule. **(E)** Here we depict an example situation where a combination of sdAbs with specificities towards the primary and the secondary antibodies are use simultaneously to extra boost the fluorescence signal. In the example both the primary and the secondary antibodies are coupled to a fluorophore, however, this is only to show that this is possible, but not strictly required.

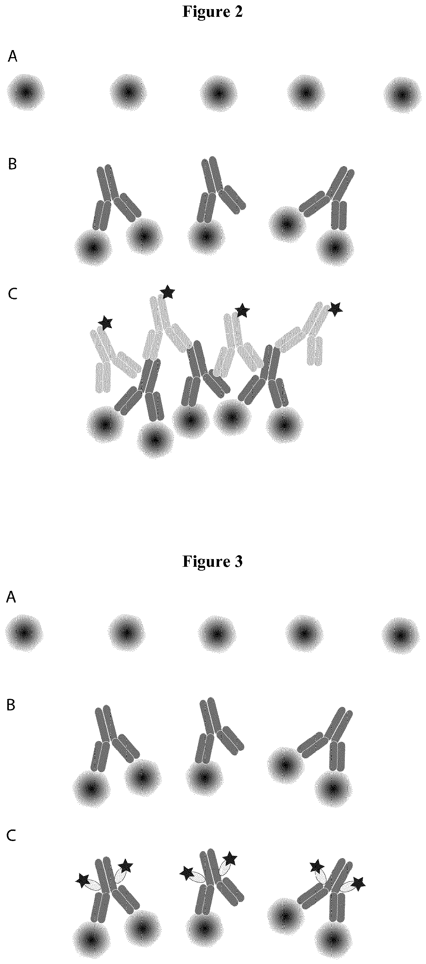

**Figure 2****:** Detection of a target using conventional antibodies (scheme). (A) Targets in their original location/conformation. (B) After primary antibodies bind to the target some rearrangement can occur. (C) Due to their divalent nature, conventional secondary antibodies enforce clustering of primary antibodies. The distribution and localization of the resulting signal may therefore differ from the true localization of the target proteins. This effect is even more pronounced if a polyclonal secondary antibody is used. Further applying tertiary antibodies will induce additional bridges between neighboring molecules that thus induce additional clustering.

**Figure 3****:** Detection of a target using and monovalent antibodies (scheme). (A) Targets in their original location/conformation. (B) After primary antibodies bind to the target some rearrangement can occur. (C) Monovalent secondary antibodies do not modify the location of the primary antibodies. Note that monovalent antibodies can directly recognize a primary antibody and that pre-mixing primary antibodies with labeled monovalent antibodies would not have any negative effects (contrary of pre-mixing conventional primary antibodies with conventional secondary antibodies).

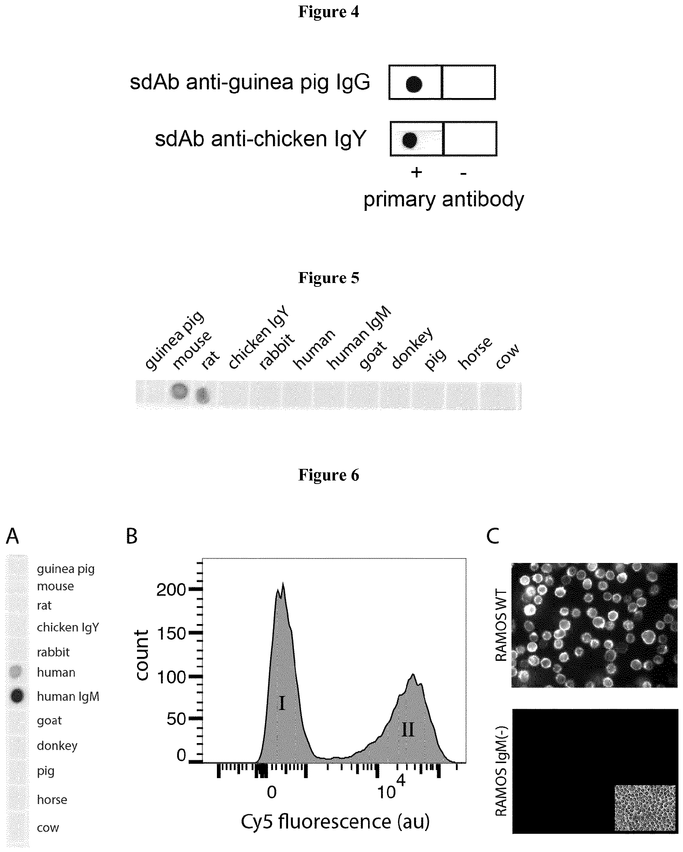

**Figure 4****:** Examples of sdAbs recognizing species-specific immunoglobulins. Upper panel: Identical amounts of a MAP2 protein fragment were spotted on two nitrocellulose membranes. The left membrane was incubated with a guinea pig antibody specifically recognizing the MAP2 protein fragment (+) while the right membrane was left untreated (-). After excessive washing, both membranes were incubated with a Cy5-labeled sdAb specifically recognizing guinea pig IgGs. Fluorescence signals (black) were acquired using a fluorescence reader (Fujifilm LAS-4000 Mini). Lower panel: An analogous experiment performed using an anti-MAP2 primary antibody raised in chicken and a Cy5-labeled sdAb directed against chicken IgY.

**Figure 5****:** Native immunoglobulins (IgGs if not stated differently) from various species were spotted on a nitrocellulose membrane (∼0.5 µg each), followed by the incubation with an sdAb bearing a 3xFLAG-tag and a GFP derivative on it C-terminus. Chemoluminescent detection was performed using an HRP conjugated anti-FLAG-tag antibody (clone M2, **F3165,** Sigma-Aldrich), developed with ECL-Plus Kit (NEL104001EA, PerkinElmer) and imaged using a Fujifilm LAS-4000 Mini. Note that the sdAb selectively recognized only the mouse and rat IgGs.

**Figure 6****:** Testing an anti-human IgM sdAb. **(A)** Specificity of an sdAb mainly recognizing human IgM. Human IgGs are recognized only weakly. **(B)** The human B-lymphocyte RAMOS cell line was stained using Cy5 coupled sdAb anti-human IgM and detected using a cell sorter (FACS). The experiment allowed to separate RAMOS cell that do not express IgMs on their surface (IgM(-); population I) from RAMOS cells expressing IgM on their surface (population II). **(C)** Wild type (WT) and IgM(-) RAMOS cells were live-stained with sdAbs coupled with Cy5 and imaged under an epifluorescence microscope. Both images were acquired under the same conditions and are displayed equally scaled for direct comparison. The bright field insert image on the bottom right corner of the IgM(-) image shows the presence of cells in the field of view.

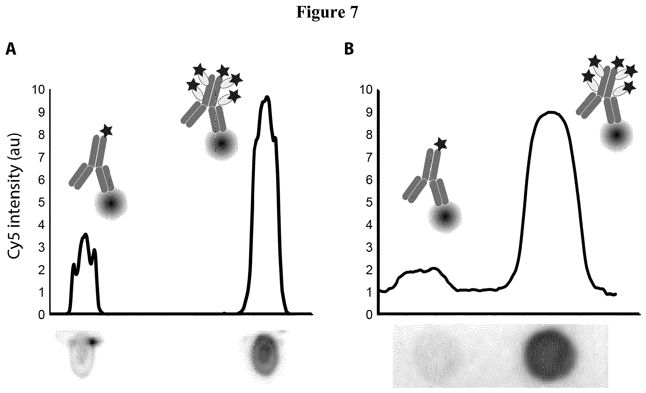

**Figure 7****:** Enhancing the signal of a fluorescently labeled immunoglobulin. **(A)** 500 µg each of parvalbumin was immobilized in two separate spots on a nitrocellulose membrane and incubated with a chicken antibody directly coupled to the fluorophore Atto647N. The left spot was detected without further incubation while the protein spotted on the right side was further incubated with an anti-chicken sdAbs coupled to Cy5. Graphs show intensity line scans along the horizontal axis together with schematic illustrations of the detected complexes. **(B)** Detection of AiF using a similar setup as in **A.** Here, AiF was detected by either an Atto647N-labeled antibody raised in guinea pig alone (left spot) or after boosting/enhancing the signal with a Cy5-labeled sdAb directed against guinea pig IgGs. Note that Atto647N and Cy5 are different molecules, but both have an equivalent excitation/emission spectrum.

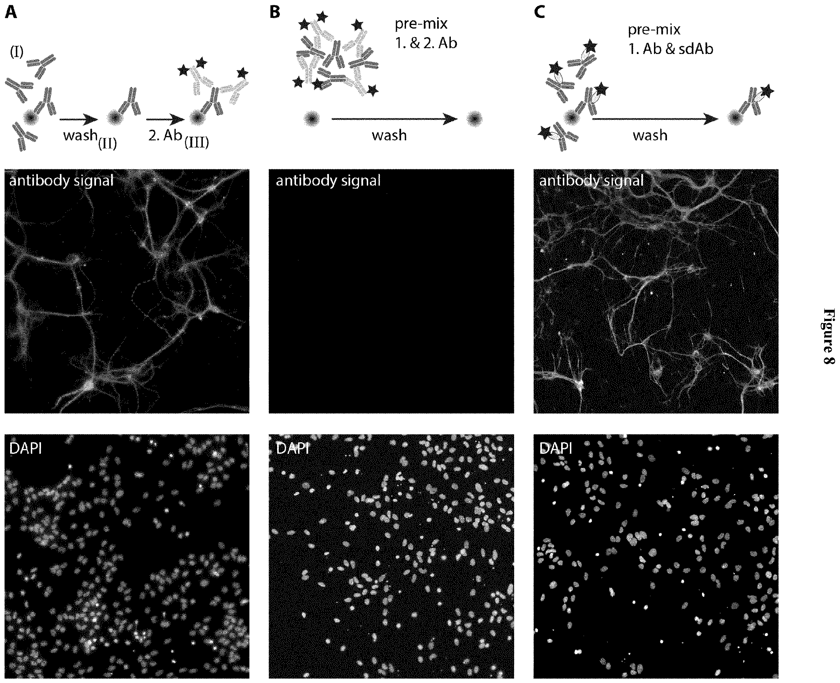

**Figure 8****:** sdAbs allow for pre-mixing of primary antibodies with sdAbs and secondary reagent and thus significantly shorten the time required for IF staining. (A) Top: Scheme depicting the basic steps in a conventional indirect immunofluorescence (IF) staining. (I) Incubation of target with primary antibody (1. Ab); typically between 0.5 and 24 h. (II) Washing the excess of 1. Ab. (III) Incubation of fluorescently labeled secondary antibody (2. Ab); typically between 0.5 and 18 h. Finally, wash off excess of 2. Ab and imaging. Middle: Example of a primary rat hippocampal neurons stained using a chicken antibody against the endogenous neuronal target β3-Tubulin (#302306 from Synaptic Systems GmbH, Göttingen Germany), detected with a conventional polyclonal anti-chicken 2. Ab coupled to Cy5 (#111-175-144 from Dianova GmbH, Hamburg Germany). Bottom: Nuclei of all (neuronal and glia) cells present in the field of view. **(B)** Ideally, to save time, the experimenter would pre-mix the 1. Ab with the 2. Ab to perform a single incubation and washing step before imaging. However, as displayed in the first image, the staining is not successful due to the clustering of 1. Ab and 2. Ab as suggested in the scheme. (C) Pre-mixing of a 1. Ab with a fluorescent monovalent secondary sdAb does not impair the specificity or quality of the staining. This will shorten the procedure for the immunostaining of cells and will avoid the potential clustering effect that can occur during a conventional indirect IF. This effect is illustrated in more detail in Figures 2 and 3.

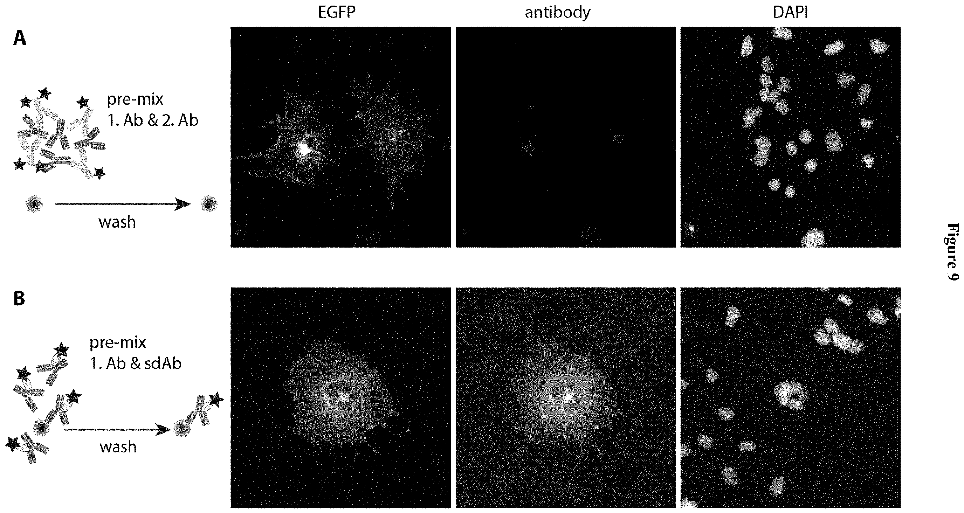

**Figure 9****:** Immunofluorescence with primary antibody premixed with secondary conventional or monovalent antibodies **(A).** A guinea pig antibody (GP) anti-EGFP was pre-mixed with a donkey anti-GP antibody coupled to Cy5 (#706-175-148 from Dianova GmbH, Hamburg Germany) and then incubated with PFA-fixed COS-7 cells transiently expressing Syntaxin1-EGFP. No antibody signal (Cy5) was visible in EGFP positive cells. Nuclear DAPI staining confirms the presence of several cells in the field of view. **(B)** Pre-mixing of the GP anti-EGFP antibody with Cy5-labeled anti-GP IgG sdAbs resulted in a clear antibody signal (Cy5) specifically on an EGFP expressing cells, but not in the EGFP-negative cells. The DAPI nuclear staining confirms that not-transfected cells were present in the imaged field of view.

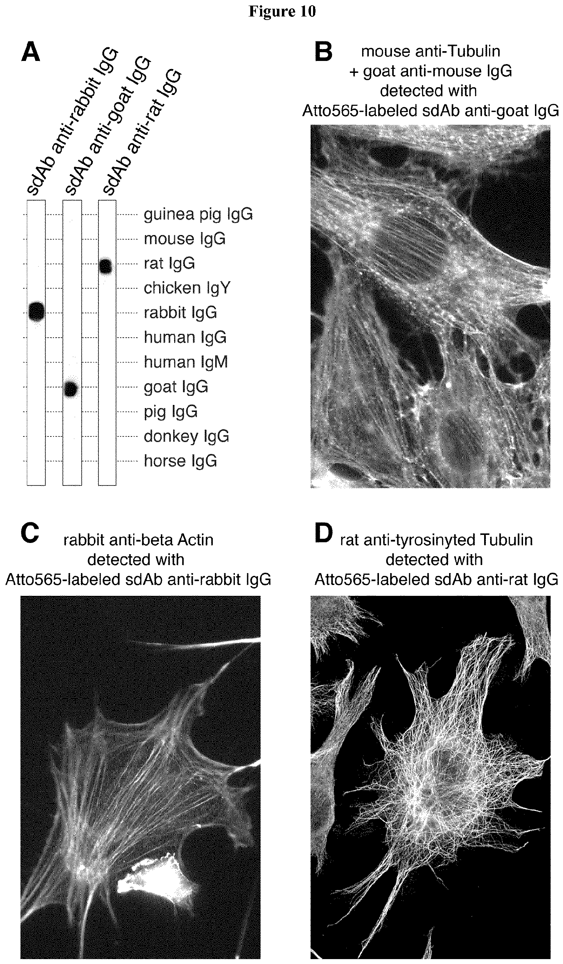

**Figure 10****: Examples for camelid single domain antibodies (sdAbs) recognizing rabbit, goat and rat IgG, respectively. (A)** Specificity of sdAbs recognizing rabbit, goat and rat IgGs, respectively. Note that all three sdAbs are specific as they do not recognize any of the other immunoglobulin species spotted on the membrane. **(B)** Immunofluorescence using an Atto565-labeled sdAb anti-goat IgG. 3T3 cells fixed with 4% paraformaldehyde were permeabilized and incubated with a primary monoclonal mouse anti-alpha Tubulin antibody (Synaptic Systems; #302211) followed by a secondary anti-mouse IgG antibody raised in goat (Jackson ImmunoResearch; #115-195-146). The secondary antibody was localized by epifluorescence microscopy using an Atto565-labeled camelid sdAb specific for goat IgGs. (C) Immunofluorescence using an Atto565-labeled sdAb anti-rabbit IgG. 3T3 cells fixed with 4% paraformaldehyde were permeabilized and incubated with a polyclonal rabbit anti-beta Actin antibody (Synaptic Systems; #251003). The primary antibody was localized by epifluorescence microscopy using an Atto565-labeled camelid sdAb specific for rabbit IgGs. **(D)** Immunofluorescence using an Atto565-labeled sdAb anti-rat IgG. 3T3 cells fixed with 4% paraformaldehyde were permeabilized and incubated with a monoclonal rat anti-tyrosinated alpha Tubulin antibody (Synaptic Systems; #302117). The primary antibody was localized by epifluorescence microscopy using an Atto565-labeled camelid sdAb specific for rat IgGs.

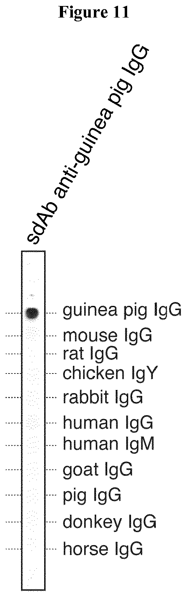

**Figure 11****:** Native immunoglobulins (IgGs if not stated differently) from various species were spotted on a nitrocellulose membrane (∼0.5 µg each), followed by the incubation with an anti-guinea pig sdAb bearing a 3xFLAG-tag and a GFP derivative on it C-terminus. Chemoluminescent detection was performed using an HRP conjugated anti-FLAG-tag antibody (clone M2, **F3165,** Sigma-Aldrich), developed with ECL-Plus Kit (NEL104001EA, PerkinElmer) and imaged using a Fujifilm LAS-4000 Mini. Note that the sdAb selectively recognized guinea pig IgG.

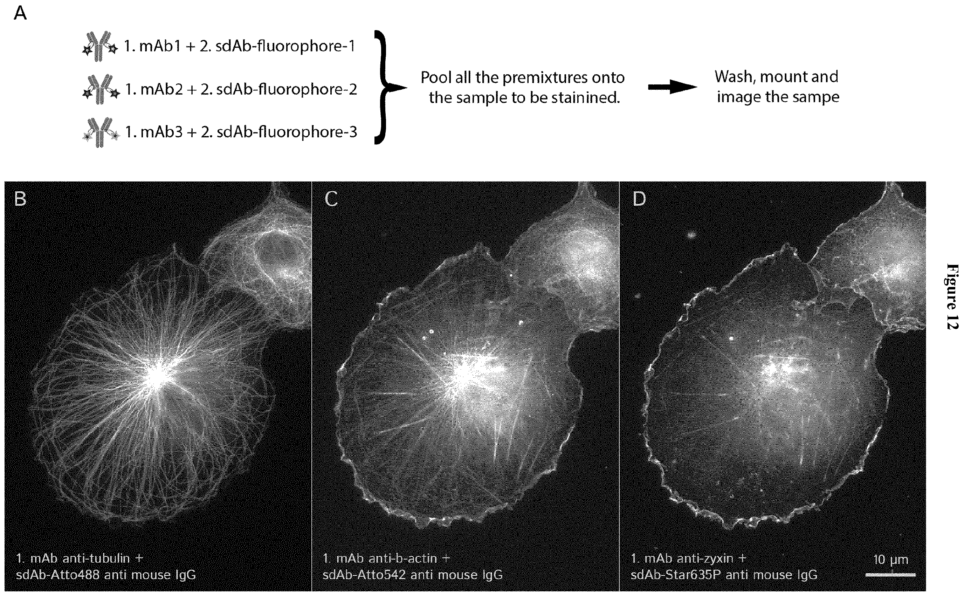

**Figure 12****:** Multiplexing immunofluorescence with several primary mouse monoclonal antibodies, each premixed with fluorescently-labeled secondary monovalent antibodies **(A).** Scheme representing the procedure: Multiplexing by premixing in separate tubes different mouse monoclonal antibodies (mAb) with monovalent antibodies coupled to different fluorophores for 1 hour. All pre-mixtures are pooled together and used for immunostaining the sample for 1 hour. After 3 washing steps the sample can be mounted and its fluorescence measured (e.g. in microscopy, FACS, western blots, etc). **(B)** A fluorescence microscopy example of multiplexing 3 mouse monoclonal primary antibodies. After pre-mixing in separate tubes i) a primary anti tubulin antibody (Cat.No. 302-211 from SySy GmbH) with a sdAb anti-mouse IgG coupled to Atto488, ii) a primary beta-Actin (Cat.No. 251-011 from SySy GmbH) with a sdAb anti-mouse IgG coupled to Atto542, and iii) an anti-zyxin (Cat.No. 307-011 from SySy GmbH) with a sdAb-coupled to AbberiorStar635P and incubation for 1h, the mixtures were simultaneously applied to the same sample, resulting in the expected specific staining pattern for each primary antibody used. Note that this type of multiplexing was achieved by pre-mixing primary and secondary antibodies, which is only possible if monovalent secondary antibodies are used, as essentially demonstrated in Fig 8 & 9.

### DETAILED DESCRIPTION OF THE INVENTION

The inventors of the present application have found that conventional methods of detecting a target antigen using a primary antibody that binds to the target antigen and a second antibody to which a detectable label is attached, suffers from several drawbacks.

In conventional methods, the secondary antibody is typically a divalent antibody, which tends to aggregate the primary antibody and thus the target. This aggregation problem is a major artifact generator for many immunofluorescence (IF) applications, especially if a high level of details and/or resolution is desired. Such artifacts are, for example, problematic when studying the quaternary structure (e.g. the multimeric state) of cellular components (e.g. of a cellular receptor). In this case, artifacts are likely to be introduced if affinity probes are used that induce dimerization or clustering of the receptor under study. This problem becomes even more evident if polyclonal secondary antibodies, which are common in the art, are used, since every primary antibody might be recognized by more than one secondary antibody, thus generating a large mesh of antibodies.

The inventors of the present application have surprisingly found that detection of target antigens can be substantially improved, if a monovalent antibody is used for detection of the primary antibody. Additionally, using a monovalent antibody as a secondary antibody avoids the clustering effect that is typically generated by conventional secondary antibodies. This is especially advantageous for applications that require high spatial resolution, e.g. for microscopy applications. Using small monovalent antibodies to detect non-labeled primary antibodies is also advantageous over using labeled primary antibodies for detection, because this completely avoids the risk of functionally inactivating the primary antibody during random conjugation to chemical fluorophores.

The inventors of the present application have further surprisingly found that spatial resolution of the detection method can further improved if a single domain antibody is used as a secondary antibody. In conventional methods, the large size of an antibody package of conventional primary and secondary antibodies, which are typically divalent full-length antibodies, displace the fluorophore from the target molecule between 15-25 nm, which is much higher than the resolution power of microscopes at the time the invention was made, which will result in an estimated error of about 10-20 nm in three-dimensional space. Using a single domain antibody as a secondary antibody may result in accuracy improvements when using high-end microscopes.

The inventors have further surprisingly found that incubation times for the detection methods can be significantly shortened by using monovalent antibodies. Current protocols, e.g. for immunofluorescence, must apply the primary antibody for a particular time (typically ranging from 0.5 to 24 h) and then wash off the excess of primary antibodies that did not find any target. After washing, the secondary antibody can be incubated for a particular time (typically ranging from 0.5 to 18 h), followed by several washing steps (typically 10 to 30 min each) to make sure no free fluorescently labeled secondary are left in the preparation. The washing steps, however, critically influence the quality of the resulting detection, which may lead to a strong background signal that hampers the detection method.

A monovalent secondary antibody can however be co-incubated with the primary antibody prior or directly on the sample. Accordingly, incubation time can be reduced to the same time needed for the conventional primary antibody step. There is no need of a further step for incubating the secondary antibody and further washing steps. This feature will hold true for various applications including immunofluorescence microscopy, immunostainings, fluorescent western blots, and cell sorting (FACS). As an illustrative example, Manning & Doe, 2017, Nature Protocols, 12(1): 1-14, discloses a method that follows conventional antibody staining protocols. In Fig.1 of Manning and Doe, it can be seen that the "new larval protocol" comprises steps of incubating the primary antibody for 3 days, rinsing for 1 day, incubating the secondary antibody for 2 days, and a final rinse for 1-3 days. Following methods of the present invention, where the monovalent secondary antibody is co-incubated with the primary antibody prior to contacting the primary antibody to the target, the incubation step of the second antibody and a second rinsing step can possibly be omitted, which may reduce incubation times of the protocol by about 4 days.

It needs to be noted that pre-incubating or co-incubating primary antibodies with conventional secondary antibodies is hardly feasible because conventional secondary antibodies will agglutinate the primary antibodies due to the conventional secondary antibodies' divalent and/or polyclonal nature.

Following the same inventive concept, the inventors of the present application have further found that a labeled monovalent antibody can also be used for improving conventional detection methods using conventional primary and secondary antibodies. Labeling of conventional secondary antibodies with e.g. fluorescent molecules is typically performed by with non-directed chemistry (i.e. random). This results in that the number of label molecules attached to each single secondary antibody is not precisely known. Typically, it follows an average distribution between 0.5 to ∼3.5 label molecules per secondary antibody. This will vary from distributor-to-distributor and batch-to-batch, which makes detection methods, especially quantitative methods, not always reproducible. An average distribution of 0.5 to ∼3.5 fluorophores per secondary antibody means that some secondary antibodies have no label (meaning the target of interest will not be detected) and some have up to 5 (which will result in an overestimation of the quantity of the target). This aspect is especially important when detection and/or quantification of a low number of molecules are desired.

Labeled monovalent antibodies specific for the secondary antibody, in particular single domain antibodies, however, can be used to enhance the signal of the label of the secondary antibody or to stoichiometrically couple label (de novo) to secondary antibodies. As traditional antibodies display two-fold rotational symmetry, typically two monovalent antibodies will bind one secondary antibody, and each monovalent antibody can be specifically labeled with a defined number of labels such as one, two, three, or even more. Therefore at least 2 label molecules, sometimes even four, six or even more, will be added per secondary antibody in a very controlled manner. Single domain antibodies are particularly advantageous for this application, as their small size facilitates their accommodation in the crowded package of primary-secondary antibodies.

The present disclosure relates to a method of detecting a target antigen by optical detection, isotopic detection, or detection by electron microscopy. The method comprises: contacting a first antibody with a target antigen that said first antibody specifically binds, or with a sample suspected to comprise said target antigen, contacting the first antibody with at least one second antibody that specifically binds the first antibody, wherein the second antibody is a monovalent antibody having one or more fluorescent label(s) chromophore label(s), isotope label(s), or metal label(s) attached to it. It is understood that the second antibody preferably has a defined number of labels attached to it.

The term "optical", as used herein, preferably refers to visible light but is generally not limited to it. The term may also refer to infrared, ultraviolet and other regions of the electromagnetic spectrum. The term "detection" as used herein includes both, direct detection of a target (i.e. wherein the target is detected by a signal deriving from a target) and indirect detection of a target (i.e. wherein the target is detected by a signal that does not directly derive from the target, e.g. by a signal that derives from another molecule attached to the target).

As used herein, "isotopic detection" relates to the detection of a molecule in which one or more atoms have been replaced (i.e. "labeled") with another isotope that commonly has a detectable variation. The isotopic label can be detected by multiple means, such as their mass (e.g. by mass spectrometry, matrix-assisted laser desorption/ionization (MALDI), desorption electrospray ionization (DESI), laser-ablation inductively coupled plasma mass spectrometry (LA-ICP-MS), or secondary-ion mass spectrometry (SIMS), vibrational mode (e.g. by infrared spectroscopy), gyromagnetic ratios (e.g. by nuclear magnetic resonance), or radioactive decay (e.g. an ionization chamber or autoradiographs of gels).

Electron microscopy relates to a method of detection using an electron microscope. Types of electron microscopes include transmission electron microscope (TEM), scanning electron microscope (SEM), reflection electron microscope (REM), scanning transmission electron microscope (STEM), and Correlative Light and Electron Microscopy (CLEM).

The term "antibody" generally refers to a proteinaceous binding molecule with immunoglobulin-like functions. Typical examples of an antibody are immunoglobulins, as well as derivatives or functional fragments thereof which still retain the binding specificity. Techniques for the production of antibodies are well known in the art. The term "antibody" also includes immunoglobulins (Ig's) of different classes (i.e. IgA, IgG, IgM, IgD, IgE, IgY etc.) and subclasses (such as IgG1, lgG2 etc.), even if recombinantly produced in foreign hosts using techniques known to those skilled in the arts. Illustrative examples of an antibody are full length immunoglobulins, F_{ab} fragments, F(ab')₂, F_{V} fragments, single-chain Fv fragments (scFv), diabodies or domain antibodies (Holt LJ et al., Trends Biotechnol. 21(11), 2003, 484-490). Domain antibodies may be single domain antibodies, single variable domain antibodies or immunoglobulin single variable domain having only one variable domain, which may be VH or VL, that specifically bind an antigen or epitope independently of other V regions or domains. A particularly preferred single domain antibody is a VHH domain heavy chain only antibody. Such an immunoglobulin single variable domain may not only encompass an isolated antibody single variable domain polypeptide, but also a larger polypeptide that includes or consists of one or more monomers of an antibody single variable domain polypeptide sequence. It is understood that a single domain antibody may comprise a VHH domain and a fusion partner, such as a protein or peptide tag. The definition of the term "antibody" thus also includes chimeric, single chain and humanized antibodies. The term "antibody" may also include fragments of antibodies.

Single domain antibodies are antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, cattle. According to the invention, a single domain antibody as used herein is derived from a naturally occurring antibody known as heavy chain antibody devoid of light chains. Such single domain antibodies are disclosed in WO 94/04678 for example. For clarity reasons, this variable domain derived from a heavy chain antibody naturally devoid of light chain is known herein as a VHH to distinguish it from the conventional VH of four chain immunoglobulins. Such a VHH molecule can be derived from antibodies raised in Camelidae species, for example in camel, dromedary, llama, vicuña, alpaca and guanaco. Other species besides Camelidae may produce heavy chain antibodies naturally devoid of light chain. As an illustrative example, it is known that sharks produce heavy chain antibodies naturally devoid of light chains (commonly named IgNAR), which also comprise a VHH domain. In addition, VHHs may be obtained from synthetic libraries. All such VHHs are within the scope of the disclosure.

VHHs, according to the present disclosure, and as known to the skilled addressee are heavy chain variable domains derived from immunoglobulins naturally devoid of light chains such as those derived from Camelidae as described in WO 94/04678 (and referred to hereinafter as VHH domains or nanobodies). VHH molecules are about 10x smaller than IgG molecules. They are single polypeptides and very stable, resisting extreme pH and temperature conditions. Moreover, they are highly resistant to the action of proteases, which is not the case for conventional antibodies. Furthermore, *in vitro* expression of VHHs or expression in prokaryotic or eukaryotic organisms suited for recombinant protein expression produces high yield, properly folded functional VHHs. It is understood that single domain antibody according to the invention is a VHH.

An antibody as used in the invention may carry one or more domains that have a sequence with at least about 60 %, at least about 70 %, at least about 75 %, at least about 80 %, at least about 85 %, at least about 90 %, at least about 92 %, at least about 95 %, at least about 96 %, at least about 97 %, at least about 98 % or at least about 99 % sequence identity with a corresponding naturally occurring domain of an immunoglobulin M, an immunoglobulin G, an immunoglobulin A, an immunoglobulin D, an immunoglobulin E , or a immunoglobulin Y. It is noted in this regard, the term "about" or "approximately" as used herein means within a deviation of 20%, such as within a deviation of 10% or within 5% of a given value or range.

Accordingly, the main chain (longer polypeptide chain) of an antibody may include, including consist of, domains with the above sequence identity with a corresponding domain of an immunoglobulin mu heavy chain, of an immunoglobulin gamma heavy chain, of an immunoglobulin alpha heavy chain, of an immunoglobulin delta heavy chain or of an immunoglobulin epsilon heavy chain. Further, an antibody molecule as used in the invention may include, including consist of, domains with the above sequence identity with a corresponding domain of an immunoglobulin lambda light chain or of an immunoglobulin kappa light chain. The entire heavy chain domains of an antibody molecule may have at least about 60 %, at least about 70 %, at least about 75 %, at least about 80 %, at least about 85 %, at least about 90 %, at least about 92 %, at least about 95 %, at least about 97 %, at least about 98 % or at least about 99 % sequence identity with the corresponding regions of an immunoglobulin mu heavy chain, of an immunoglobulin gamma heavy chain (such as gamma 1, gamma 2, gamma 3 or gamma 4 heavy chains), of an immunoglobulin alpha heavy chain (such as alpha 1 or alpha 2 heavy chains), of an immunoglobulin delta heavy chain or of an immunoglobulin epsilon heavy chain. A light chain domains present in an antibody may have at least about 60 %, at least about 70 %, at least about 75 %, at least about 80 %, at least about 85 %, at least about 90 %, at least about 92 %, at least about 95 %, at least about 97 %, at least about 98 % or at least about 99 % sequence identity with the corresponding regions of an immunoglobulin lambda light chain (such as lambda 1, lambda 2, lambda 3 or lambda 4 light chains) or of an immunoglobulin kappa light chain.

"Percent (%) sequence identity" with respect to amino acid sequences disclosed herein is defined as the percentage of amino acid residues in a candidate sequence that are pair-wise identical with the amino acid residues in a reference sequence, i.e. an antibody molecule of the present disclosure, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The same is true for nucleotide sequences disclosed herein.

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable regions", "HVR," or "HV," or "complementarity determining regions" (CDRs). The more conserved (i.e., non-hypervariable) portions of the variable domains are called the "framework" regions (FR). The variable domains of naturally occurring heavy and light chains each include four FR regions, largely adopting a β-sheet configuration, connected by three hypervariable regions, which form loops connecting, and in some cases forming part of, the β -sheet structure. The hypervariable regions in each chain are held together in close proximity by the FR and, with the hypervariable regions from the other chain, contribute to the formation of the antigen-binding site (see Kabat et al., see below). Generally, naturally occurring immunoglobulins include six CDRs (see below); three in the VH (H1, H2, H3), and three in the VL (L1, L2, L3). In naturally occurring immunoglobulins, H3 and L3 display the most diversity of the six CDRs, and H3 in particular is believed to play a unique role in conferring fine specificity to immunoglobulins. The constant domains are not directly involved in antigen binding, but exhibit various effector functions, such as, for example, antibody-dependent, cell-mediated cytotoxicity and complement activation.

The corresponding immunoglobulin mu heavy chain, gamma heavy chain, alpha heavy chain, delta heavy chain, epsilon heavy chain, lambda light chain or kappa light chain may be of any species, such as a mammalian species, including a rodent species, an avian species, an amphibian species, or an invertebrate species. Examples of mammals include, but are not limited to, a rat, a mouse, a rabbit, a guinea pig, a donkey, a sheep, a squirrel, a hamster, a hedgehog, a platypus, an American pika, an armadillo, a dog, a lemur, a goat, a pig, a cattle, an opossum, a horse, a bat, a woodchuck, an orang-utan, a rhesus monkey, a woolly monkey, a macaque, a chimpanzee, a tamarin (saguinus oedipus), a marmoset or a human. Examples of avians include, but are not limited to, a chicken.

The term "immunoglobulin" refers to a glycoprotein that may include at least two heavy (H) chains and two light (L) chains linked by disulfide bonds, or an antigen binding portion thereof. Each heavy chain has a heavy chain variable region (abbreviated herein as V_{H}) and a heavy chain constant region. The heavy chain constant region may include three domains, C_{H1}, C_{H2} and C_{H3}. Each light chain has a light chain variable region (abbreviated herein as V_{L}) and a light chain constant region. The light chain constant region includes one domain, C_{L}. The V_{H} and V_{L} regions can be further subdivided into regions of hypervariability, termed complementarity determining regions (CDR), interspersed with regions that are more conserved, termed framework regions (FR). The CDRs contain most of the residues responsible for specific interactions of the antibody with the antigen. Each V_{H} and V_{L} has three CDRs and four FRs, arranged from amino-terminus (N-terminus) to carboxy-terminus (C-terminus) in the following order: FR1, CDR1, FR2, CDR2, FR3, CDR3, FR4. The variable regions of the heavy and light chains contain a binding domain that interacts with an epitope of an antigen. The term "immunoglobulin" may also include two heavy chains without light chains, such as e.g. an antibody devoid of light chains.

Each light chain of an immunoglobulin includes an N-terminal variable (V) domain (VL) and a constant (C) domain (CL). Each heavy chain includes an N-terminal V domain (VH), three or four C domains (CHs), and a hinge region.

An immunoglobulin when used herein, may be a tetrameric glycosylated protein composed of two light (L) chains of approximately 25 kDa each and two heavy (H) chains of approximately 50 kDa each. Two types of light chain, termed lambda and kappa, may be found in immunoglobulins. Depending on the amino acid sequence of the constant domain of heavy chains, immunoglobulins can be assigned to five major classes: A, D, E, G, M, and Y, and several of these may be further divided into subclasses (isotypes), e.g., IgG1, lgG2, IgG3, IgG4, IgA1, and IgA2. An IgM immunoglobulin consists of 5 of the basic heterotetramer unit along with an additional polypeptide called a J chain, and contains 10 antigen binding sites, while IgA immunoglobulins contain from 2-5 of the basic 4-chain units which can polymerize to form polyvalent assemblages in combination with the J chain. In the case of IgGs, the 4-chain unit is generally about 150,000 Daltons. The term "amino acid" or "amino acid residue" refers to an α- or β-amino carboxylic acid. An immunoglobulin when used herein may also be a dimeric glycosylated protein composed of two heavy chains such as a camelid heavy chain only IgG (hcIgG) or a shark IgNAR.

When used in connection with a protein or peptide, the term "amino acid" or "amino acid residue" typically refers to an α-amino carboxylic acid having its art recognized definition such as an amino acid selected from the group consisting of: L-alanine (Ala or A); L-arginine (Arg or R); L-asparagine (Asn or N); L-aspartic acid (Asp or D); L-cysteine (Cys or C); L-glutamine (Gln or Q); L-glutamic acid (Glu or E); glycine (Gly or G); L-histidine (His or H); L-isoleucine (Ile or I): L-leucine (Leu or L); L-lysine (Lys or K); L-methionine (Met or M); L-phenylalanine (Phe or F); L-proline (Pro or P); L-serine (Ser or S); L-threonine (Thr or T); L-tryptophan (Trp or W); L-tyrosine (Tyr or Y); and L-valine (Val or V), although modified, synthetic, or rare amino acids such as e.g. taurine, ornithine, selenocysteine, homocystine, hydroxyproline, thioproline, iodo-tyrosine, 3-nitro-tyrosine, ornithine, citrulline, canavanine, 5-hydroxytryptophane, carnosine, cycloleucine, 3,4-dihydroxy phenylalanine, N-acetylcysteine, prolinol, allylglycine or acetidine-2-carboxylic acid may be used as desired. Generally, amino acids can be grouped as having a nonpolar side chain (e.g., Ala, Cys, Ile, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

A target is any substance of biological or chemical origin to which an antibody as used in the invention is capable to detect directly or indirectly. Targets may be, for example, proteins, peptides, nucleic acids, oligonucleic acids, saccharides, polysaccharides, glycoproteins. Examples include, but are not limited to therapeutic targets, diagnostic targets, receptors, receptor ligands, viral coat proteins, immune system proteins, hormones, enzymes, antigens, cell signaling proteins, or a fragment thereof. Targets may be native protein or a fragment thereof, a homologous sequence thereof, a functional portion thereof, or a functional portion of a homologous sequence.

The term "epitope", also known as the "antigenic determinant", refers to the portion of an antigen to which an antibody specifically binds, thereby forming a complex. Thus, the term "epitope" includes any molecule or protein determinant capable of specific binding to an immunoglobulin or T-cell receptor. The binding site(s) (paratope) of an antibody molecule described herein may specifically bind to/interact with conformational or continuous epitopes, which are unique for the target structure. Epitopic determinants usually consist of chemically active surface groupings of molecules such as amino acids or sugar side chains and usually have specific three dimensional structural characteristics, as well as specific charge characteristics. Epitope determinants may include chemically active surface groupings of molecules such as amino acids, sugar side chains, phosphoryl, or sulfonyl, and, in certain embodiments, may have specific three-dimensional structural characteristics, and/or specific charge characteristics. With regard to polypeptide antigens a conformational or discontinuous epitope is characterized by the presence of two or more discrete amino acid residues, separated in the primary sequence, but assembling to a consistent structure on the surface of the molecule when the polypeptide folds into the native protein/antigen (Sela, M., Science (1969) 166, 1365-1374; Laver, W.G., et al. Cell (1990) 61, 553-556). The two or more discrete amino acid residues contributing to the epitope may be present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain. As an illustrative example, a "context-dependent" CD3 epitope refers to the conformation of said epitope. Such a context-dependent epitope, localized on the epsilon chain of CD3, can only develop its correct conformation if it is embedded within the rest of the epsilon chain and held in the right position by heterodimerization of the epsilon chain with either CD3 gamma or delta chain. In contrast thereto, a context-independent CD3 epitope may be an N-terminal 1-27 amino acid residue polypeptide or a functional fragment thereof of CD3 epsilon. Generally, epitopes can be linear in nature or can be a discontinuous epitope. Thus, as used herein, the term "conformational epitope" refers to a discontinuous epitope formed by a spatial relationship between amino acids of an antigen other than an unbroken series of amino acids. The term "epitope" also includes an antigenic determinant of a hapten, which is known as a small molecule that can serve as an antigen by displaying one or more immunologically recognized epitopes upon binding to larger matter such as a larger molecule e.g. a protein.

An antibody or antibody molecule/fragment is said to "specifically" bind to an antigen when it recognizes its target antigen within a complex mixture of proteins and/or macromolecules. Typically, the antibody is capable of specifically interacting with and/or binding to its target but does not essentially bind to another epitope or antigen. Antibodies are said to "bind to the same epitope" if the antibodies cross-compete so that only one antibody can bind to the epitope at a given point of time, i.e. one antibody prevents the binding or modulating effect of the other.

Typically, binding that is considered specific may also have a high affinity, e.g. when the binding affinity is higher than 10⁻⁶ M (in terms of K_{D}). In particular, the binding affinity may be about 10⁻⁸ to 10⁻¹¹ M (K_{D}), or of about 10⁻⁹ to 10⁻¹¹ M or even higher. Thus, antibody molecules with an affinity in the picomolar range (with a K_{D} of 9.9x10⁻¹⁰ M to 10⁻¹² M) are also encompassed in the present invention. If necessary, nonspecific binding of a binding site can be reduced without substantially affecting specific binding by varying the binding conditions.

An antigen to which an antibody used in the invention binds may be an antigen that is included in the extracellular matrix or it may a cell surface antigen, or it may be an antigen located inside a cell. An antigen to which an antibody used in the invention binds may also be an immunoglobulin.

An antibody according to the disclosure may be an isolated antibody molecule. The term "isolated antibody molecule" as used herein refers to an antibody molecule that has been identified and separated and/or recovered from a component of its natural environment. Contaminant components of its natural environment are matter that would interfere with diagnostic or therapeutic uses for the antibody, and may include enzymes, hormones, and other proteinaceous or non-proteinaceous solutes. In some examples the antibody molecule is purified to greater than 95% by weight of antibody as determined by the Lowry method, such as more than 99% by weight. In some examples the antibody molecule is purified to a degree sufficient to obtain at least 15 residues of N-terminal or internal amino acid sequence by use of a spinning cup sequenator. In some examples the antibody is purified to homogeneity as judged by SDS-PAGE under reducing or nonreducing conditions using Coomassie blue or, preferably, silver stain. An isolated antibody molecule may in some examples be present within foreign host cells with one or more component(s) of the antibody's natural environment not being present. Typically an isolated antibody is prepared by at least one purification step.

Unless otherwise indicated CDRs sequences of the disclosure follow the definition by Maass 2007 (Journal of Immunological Methods 324 (2007) 13-25). Other standards for defining CDRs exist as well, such as the definition according to Kabat CDRs, as described in Sequences of Proteins of immunological Interest, US Department of Health and Human Services (1991), eds. Kabat et al. Another standard for characterizing the antigen binding site is to refer to the hypervariable loops as described by Chothia (see, e.g., Chothia, et al. (1992); J. Mol. Biol. 227:799-817; and Tomlinson et al. (1995) EMBO J. 14:4628-4638). Still another standard is the AbM definition used by Oxford Molecular's AbM antibody modelling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). It is understood that embodiments described with respect to the CDR definition of Maass, can alternatively be implemented using similar described relationships such as with respect to Kabat CDRs, Chothia hypervariable loops or to the AbM-defined loops.

The valence of an antibody is generally an expression of the number of antigen-binding sites for one molecule of any given antibody or the number of antibody-binding sites for any given antigen. Most antibody molecules, and those belonging to the IgG, IgA, IgD and IgE immunoglobulin classes, have two antigen-binding sites per molecule. In general, a monovalent antibody comprises a single antigen binding site. Examples for a monovalent antibody are a single domain antibody, a VHH domain, a single Fab fragment, a Fv fragment, a scFv fragment, or a single VH or VL domain.

The terms "Fab", "Fab region", "Fab portion" or "Fab fragment" are understood to define a polypeptide that includes a V_{H}, a C_{H}1, a V_{L}, and a C_{L} immunoglobulin domain. Fab may refer to this region in isolation, or this region in the context of an antibody molecule according to the disclosure, as well as a full-length immunoglobulin or immunoglobulin fragment. Typically a Fab region contains an entire light chain of an antibody. A Fab region can be taken to define "an arm" of an immunoglobulin molecule. It contains the epitope-binding portion of that Ig. The Fab region of a naturally occurring immunoglobulin can be obtained as a proteolytic fragment by a partial papain-digestion. A "F(ab')₂ portion" is the proteolytic fragment of a partially pepsin-digested immunoglobulin. A "Fab' portion" is the product resulting from reducing the disulfide bonds of an F(ab')₂ portion. As used herein the terms "Fab", "Fab region", "Fab portion" or "Fab fragment" may further include a hinge region that defines the C-terminal end of the antibody arm (cf. above). This hinge region corresponds to the hinge region found C-terminally of the C_{H}1 domain within a full length immunoglobulin at which the arms of the antibody molecule can be taken to define a Y. The term hinge region is used in the art because an immunoglobulin has some flexibility at this region.

An "Fv" or "Fv fragment" consists of only the V_{L} and V_{H} domains of a "single arm" of an immunoglobulin. Thus an "Fv" is the minimum antibody fragment which contains a complete antigen-recognition and binding site. A "two-chain" Fv fragment consists of a dimer of one heavy- and one light-chain variable domain in tight, non-covalent association. A single-chain Fv species (scFv) includes a V_{H} and a V_{L} domain of an immunoglobulin, with these domains being present in a single polypeptide chain in which they are covalently linked to each other by a flexible peptide linker. Typically, in a scFv fragment the variable domains of the light and heavy chain associate in a dimeric structure analogous to that in a two-chain Fv species. In single chain Fv fragments, it is possible to either have the variable domain of the light chain arranged at the N-terminus of the single polypeptide chain, followed by the linker and the variable domain of the heavy chain arranged at the C-terminus of the polypeptide chain or vice versa, having the variable domain of the heavy chain arranged on the N-terminus and the variable domain of the light chain at the C-terminus with the peptide linker arranged in between. The peptide linker can be any flexible linker known in the art, for example, made from glycine and serine residues. It is also possible to additionally stabilize the domain association between the V_{H} and the V_{L} domain by introducing disulfide bonds into conserved framework regions (see Reiter et al. Stabilization of the Fv fragments in recombinant immunotoxins by disulfide bonds engineered into conserved framework regions, Biochemistry 1994, 33, 6551-5459). Such scFv fragments are also known as disulfide-stabilized scFv fragments (ds-scFv).

The term "Fc region" or "Fc fragment" is used herein to define a C-terminal region of an immunoglobulin heavy chain, including native-sequence Fc regions and variant Fc regions. The Fc part mediates the effector function of antibodies, e.g. the activation of the complement system and of Fc-receptor bearing immune effector cells, such as NK cells. In human IgG molecules, the Fc region is generated by papain cleavage N-terminal to Cys226. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody molecule, or by recombinantly engineering the nucleic acid encoding a heavy chain of the antibody molecule. Accordingly, a composition of intact antibodies may include antibody populations with all K447 residues removed, antibody populations with no K447 residues removed, and antibody populations having a mixture of antibodies with and without the K447 residue. Suitable native-sequence Fc regions for use in the antibodies of the disclosure include mammalian, e.g., human or murine, IgG1, IgG2 (IgG2A, IgG2B), IgG3 and IgG4. The Fc region contains two or three constant domains, depending on the class of the antibody. In examples where the immunoglobulin is an IgG the Fc region has a C_{H}2 and a C_{H}3 domain.

An antibody used in the invention may be produced using any known and well-established expression system and recombinant cell culturing technology, for example, by expression in bacterial hosts (prokaryotic systems), or eukaryotic systems such as yeasts, fungi, insect cells or mammalian cells. An antibody molecule according to the present invention may be produced in transgenic organisms such as a goat or a plant. An antibody may also be produced by chemical synthesis.

It is also possible to equip one of the chains of an antibody with an affinity tag. Affinity tags such as Strep-tag® or Strep-tag® II (Schmidt, T.G.M. et al. (1996) J. Mol. Biol. 255, 753-766), myc-tag, FLAG™-tag, His6-tag, HA-tag, MBP-tag, GST-tag, SUMO-tag, and fluorescent tags such as GFP tag (and derivatives), or dsRed (and derivatives) allow easy detection and also simple purification of the recombinant antibody molecule.

A method of the invention comprises the use of two antibodies for the detection of a target antigen, as defined in the appended claims. The first (primary), preferably unlabeled, antibody specifically binds to the target antigen. The second (secondary) antibody, which carries a fluorescent label, recognizes the primary antibody and binds to it. Here, one or more secondary antibodies can bind a single primary antibody. According to the invention, two identical monovalent secondary antibodies typically bind to one first antibody. This provides signal amplification by increasing the number of detectable labels per antigen.

First antibodies with different constant regions may typically be generated by raising the antibody in different species. For example, it is possible to use a first antibody that has been created in one species and recognizes the target antigen, and then employ a secondary antibody that carries the detectable label and recognizes the first antibody, preferably at the constant region. Optionally, it is possible to use one or more further primary antibodies that have been created in one or more further (different) species that recognize one or more further target antigens, and then employ further one or more secondary antibodies that carry one or more further detectable labels that are distinguishable from the first (or further) detectable labels that recognize the antibody constant region of the one or more further first antibodies.

One or more of the antibodies of the disclosure has a detectable label attached to it. There are many types of detectable labels, including a fluorescent label, a chromophore label, an isotope label, or a metal label, with a fluorescent label being preferred. The presence of the target antigen can be detected by detecting the signal of the detectable label. For a fluorescent label, this means detection of emitted light upon excitation of the fluorescent label. Non-exhaustive examples for suitable fluorescent labels are "green" emitters (Atto488, Alexa488, Cy2, etc), "orange" emitters (Atto542, alexa555, Cy3, etc), "Red-far-Red" emitters (Alexa633, Atto 647N, Cy5, etc), infrared emitters (Atto700, LiCor IRDye700, LiCor IRDye800, etc.), ultra-violet absorbing fluorescent dyes (Atto390 or Alexa405). Non-exhaustive examples for a suitable chromophore label are alkaline phosphatase or peroxidase exposed to TMB (3,3',5,5' tetramethylbenzidine), DAB (3,3',4,4' diaminobenzidine), and 4CN (4-chloro-1-naphthol). ABTS (2,2'-azino-di [3-ethyl- benzthiazoline] sulfonate), OPD (o-phenylenediamine), and to BCIP/NBT (5-bromo- 4-chloro-3-indolyl-phosphate/nitroblue tetrazolium). Non-exhaustive examples for isotope labels are 13C, 15N, 19F, 27A1, 11B, 1271 or different Lanthanides isotopes. Non-exhaustive examples for a metal label are Au, Pd, Pb, Pt Ag, Hg and Os.

According to the methods of the present invention, the steps of contacting the first antibody with the target antigen and contacting the second monovalent antibody with the first antibody can be carried out in any order. Here, contacting the second monovalent antibody with the first antibody in a first step will have several advantages and is thus preferred, since the resulting complex of first antibody and second antibody can be directly applied onto the sample comprising or suspected to comprise the target antigen. The step of contacting first and second antibody can be conducted prior to or at the same time as preparation of the sample comprising or suspected to comprise the target antigen, which means that the time spanning from sample preparation to detection of the target antigen can be significantly reduced. This is possible only because a monovalent second antibody is used, as a divalent or multivalent second antibody would form aggregates with the first antibody. Following this inventive concept, it is also possible to premix one first antibody with one second antibody, which will result in one complex of the one first antibody and the one second antibody, and to premix another first antibody with another second antibody, which will result in another complex of the another first antibody and the another second antibody. The one complex of the one first antibody and the one second antibody may then be mixed with the another complex of the another first antibody and the another second antibody, and the mixture may then be applied on the sample. In cases where the one first antibody and the another first antibody have different specificities and both specific targets are present in the sample, two different targets can be stained and/or labeled in one single step. In such a case, the label of the one second antibody and the another second antibody are preferably distinguishable. It is also possible to add one or more further (preferably premixed) complex(es) of first and second antibody to the mixture of the one first and second antibody complex and the another first and second antibody complex. The resulting mixture preferably comprises all three or more complexes of first and second antibodies and may be applied onto the target. By doing so, three or even more targets can be stained and/or labeled in one step.

Alternatively, contacting the first antibody with the target antigen and contacting the second monovalent antibody with the first antibody can preferably be carried out in one single step. Here again, the time spanning from sample preparation to detection of the target antigen can be significantly reduced since residual primary and secondary antibodies can be washed off simultaneously, thereby reducing washing steps. This is only possible because a monovalent second antibody is used, as a divalent or multivalent second antibody would again form aggregates with the first antibody.

The second monovalent antibody may preferably be or comprise a single domain antibody. It may typically be a single-domain antibody from a camelid or a shark, with a camelid single domain antibody being preferred. It is understood "single domain antibody from a camelid or a shark" or a "camelid or shark single domain antibody" as used herein typically refers to a VHH fragment of a camelid or shark antibody devoid of light chains. Said VHH fragments are typically obtained by recombinant expression. In the invention, the second antibody is a camelid single domain antibody as defined in the appended independent claims. The second monovalent antibody may be polyclonal or monoclonal with monoclonal being preferred. It is also possible to employ two or more different second monovalent antibodies that specifically recognize the same first antibody but that do not cross-compete with each other. This can be achieved if the two or more second antibodies bind to different epitopes of the first antibody. For example, if two of said second antibodies are used, up to four second antibodies can bind in total to one first antibody. If both said second antibodies are labeled with the same or a similar detectable label, e.g. four, six, eight, or even more detectable labels will be attached to one first antibody (depending on the number of detectable labels attached to each second monovalent antibody), which provides stronger signal amplification as compared to methods, where only one second antibody is employed. It is understood that said two or more second antibodies are preferably monoclonal antibodies.

The first antibody may be any type of antibody, including an immunoglobulin, such as an immunoglobulin G (IgG), immunoglobulin A (IgA), immunoglobulin D (IgD), immunoglobulin E (IgE), immunoglobulin M (IgM), or immunoglobulin Y (IgY). It may be derived from any species, preferably a mammalian or avian species. Preferred species are e.g. rabbit, goat, donkey, guinea pig, rat, chicken, and mouse, cattle, hamster, sheep, and human. The first antibody can be polyclonal or monoclonal. It is understood that the second monovalent antibody specifically binds to an antibody of the same species as the first antibody, e.g. specifically bind to an antibody selected from the group consisting of rabbit, goat, donkey, guinea pig, rat, chicken, and mouse, cattle, hamster, sheep, and human, depending on the species of the first antibody. It is also possible that the second antibody specifically binds to the class of immunoglobulins of the first antibody, such as e.g., IgG, IgA, IgD, IgE, IgM, or IgY. Although not essential, it is in most cases preferred that the second monovalent antibody is not cross-reactive with an immunoglobulin of at least one second species, such as an immunoglobulin of another species selected from the group consisting of rabbit, goat, donkey, guinea pig, rat, chicken, and mouse, cattle, hamster, sheep, and human.

An exemplary second monovalent antibody is a camelid single domain antibody specific for guinea pig IgG. Such a second monovalent antibody may have a CDR1 sequence of GLTFSEYV (SEQ ID NO: 01), a CDR2 sequence of VAAMNSRT (SEQ ID NO: 02), and a CDR3 sequence of AVGAYGDYAGPSHFHS (SEQ ID NO: 03). As an illustrative example, such a second monovalent antibody may comprise or consist of a VHH domain that has the amino acid sequence of

Another exemplary second monovalent antibody is a camelid single domain antibody specific for chicken IgY. Such a second monovalent antibody may have a CDR1 sequence of GNISNILS (SEQ ID NO: 05), a CDR2 sequence of ITNDGNT (SEQ ID NO: 06), and a CDR3 sequence of NAARWTQPRPS (SEQ ID NO: 07). As an illustrative example, such a second monovalent antibody may comprise or consist of a VHH domain that has the amino acid sequence of

Another exemplary second monovalent antibody is a camelid single domain antibody specific for mouse and/or rat IgG. Such a second monovalent antibody may have a CDR1 sequence of GRTDSLYA (SEQ ID NO: 09), a CDR2 sequence of ITWIGGAV (SEQ ID NO: 10), and a CDR3 sequence of ASTFRRIWDAEESIFDT (SEQ ID NO: 11). As an illustrative example, such a second monovalent antibody may comprise or consist of a VHH domain that has the amino acid sequence of

Another exemplary second monovalent antibody is a camelid single domain antibody specific for human IgM, which may also recognize human IgG with a lower affinity. Such a second monovalent antibody may have a CDR1 sequence of GNSFSISN (SEQ ID NO: 13), a CDR2 sequence of ITSGGVA (SEQ ID NO: 14), and a CDR3 sequence of HCESGFPTLIAY (SEQ ID NO: 15). As an illustrative example, such a second monovalent antibody may comprise or consist of a VHH domain that has the amino acid sequence of

Further exemplary second monovalent antibodies can be generated by methods known in the art. As an illustrative example, antigen-specific camelid single domain antibodies (sdAbs) can be obtained following established protocols (see e.g. Pleiner et al. Elife. 2015 Dec 3;4:e11349. doi: 10.7554/eLife.11349 or Pardon et al. Nat Protoc. 2014 Mar; 9(3):674-93. doi: 10.1038/nprot.2014.039.). In general, in some exemplary methods , generation of camelid single domain antibodies can be divided in successive phases. In phase I (immunization), as an illustrative example, camelids are immunized 4-6 times with the respective antigen in a suitable adjuvant (e.g. Gerbu Fama or Freunds adjuvants) over a period of several weeks. Phase II (library generation): As an illustrative example, after the last immunization, peripheral blood is withdrawn, from which B-lymphocytes are extracted using established techniques. After RNA extraction and cDNA synthesis, single-domain antibody-encoding regions are selectively amplified by polymerase chain reaction (PCR) and cloned into established vectors suitably for phage display (phagemids). Phase III (screening): As an illustrative example, *Escherichia coli* with intact F pili are transformed with the resulting phagemid library and infected with suitable m13 helper phage to produce an infective phage library carrying sdAbs on their surface. Phages specifically binding the target protein are selected by iterative cycles of affinity purification, infection of *E. coli* and phage amplification. It is also possible to include negative selection steps, in which phages that bind to a non-desired second target are excluded. Phase IV (expression): Selected sdAbs are expressed in a suitable host following established protocols (see e.g. Pleiner et al. Elife. 2015 Dec 3;4:e11349. doi: 10.7554/eLife.11349).

The present disclosure also relates to a method of optically detecting a target antigen comprising: contacting a first antibody with a target antigen that said first antibody specifically binds, or with a sample suspected to comprise said target antigen contacting a first antibody with a second antibody that specifically binds the first antibody; contacting at least one third antibody that specifically binds the second antibody with the second antibody, wherein the third antibody is a monovalent antibody having one or more fluorescent label(s), chromophore label(s), an isotope label(s), or a metal label(s) attached to it, with fluorescent label(s) being preferred. It is understood that the third antibody preferably has a defined number of labels attached to it.

A method of the disclosure may thus also comprise the use of three antibodies for the detection of a target antigen. A first (primary), preferably unlabeled, antibody specifically binds to the target antigen. A second (secondary), preferably non-monovalent, antibody, which preferably carries a detectable label, recognizes the primary antibody and binds to it. The third antibody is a monovalent antibody that binds to the second, preferably non-monovalent, antibody but preferably does not bind to the first antibody. The third antibody, which may also be referred to as the "booster antibody", preferably carries a detectable label. Here, one or more booster antibodies can bind a single secondary antibody. It is also possible to use two or more different booster antibodies for binding a single secondary antibody. According to the disclosure, two identical third antibodies typically bind to one second antibody, respectively. The first, second and third antibodies are preferably generated by raising the antibodies in different species. For example, the first antibody that recognizes the target antigen has been created in a one species, and secondary antibody that recognize the first antibody, has been created in a second species. The third antibody may optionally be generated in a third species or may be recombinant or synthetic and recognizes the second antibody but preferably does not recognize the first antibody.

The method may further comprise contacting the first antibody with a fourth antibody that specifically binds the first antibody, wherein the fourth antibody is a monovalent antibody having one or more fluorescent label(s), chromophore label(s), isotope label(s), or metal label(s) attached to it. The detectable label of the fourth monovalent antibody and the third monovalent antibody may be the same or different. If the detectable labels are different, they may be similar or non-similar. The detectable labels of both the third monovalent antibody and the fourth monovalent antibody may preferably be fluorescent labels.

The detectable label of the second, preferably non-monovalent, antibody and the third (booster) antibody may be any type of detectable label disclosed herein, including a fluorescent label, a chromophore label, an isotope label, or a metal label, with fluorescent labels being preferred. The detectable labels of the second, preferably non-monovalent, antibody and the third (booster) antibody may be different from each other. However, it is preferred that the detectable labels are the same or similar. For example, the labels of the second, preferably non-monovalent, antibody and the third (booster) antibody may be the same chromophore label or the same fluorescent label. A "similar" label, as used herein refers to a label that has a similar absorption spectrum, or a similar maximum absorption and emission wavelength. In case of a fluorescent label, two "similar" labels may also have a similar emission spectrum or maximum emission wavelength. An illustrative example for two labels that are similar are Atto647N (ATTO-TEC, Germany), which has an absorption maximum at 646 nm and an emission maximum at 664 nm, and Cy5 dye, which has an absorption maximum at 649 nm an emission maximum at 670 nm. Ideally, adsorption maximum and/or emission maximum of two "similar" labels should differ in not more than about 20 nm, preferably not more than about 15 nm, preferably not more than about 10 nm, preferably not more than about 7 nm, preferably not more than about 5 nm.

The presence of the target antigen can be detected by detecting the signal of the detectable label. For a fluorescent label, this means detection of emitted light upon excitation of the fluorescent label. Examples for suitable fluorescent labels are disclosed herein.

According to the methods of the present disclosure, the steps of contacting the second, preferably non-monovalent, antibody with the first antibody and contacting the third monovalent antibody with the second, preferably non-monovalent, antibody can be carried out in any order. Here, contacting the third monovalent antibody with the second, preferably non-monovalent, antibody in a first step will have several advantages and is thus preferred, since the resulting complex of second, preferably non-monovalent, antibody and third monovalent antibody can then be directly applied onto a sample comprising or suspected to comprise the target antigen that ideally comprises a first antibody bound to the target antigen. The step of contacting second and third antibody can be conducted prior to or at the same time as preparation of the sample comprising or suspected to comprise the target antigen and comprising a first antibody bound to the target antigen, which means that the time spanning from sample preparation to detection of the target antigen can be significantly reduced compared to methods, in which the second antibody is contacted with a sample comprising the first antibody bound to the target antigen prior to contacting the second antibody with the third antibody. The step of contacting the first antibody with a target antigen that the first antibody specifically binds, or with a sample suspected to comprise said target antigen should however be conducted prior to contacting the first antibody with the second, preferably non-monovalent, antibody that specifically binds the first antibody.

Alternatively, contacting the second, preferably non-monovalent, antibody with the first antibody that is bound to the target antigen and contacting the third monovalent antibody with the second antibody can preferably be carried out in one single step. Here again, compared to methods, in which the second antibody is contacted with a sample comprising the first antibody bound to the target antigen prior to contacting the second antibody with the third antibody, the time spanning from sample preparation to detection of the target antigen can be significantly reduced since residual second and third antibodies can be washed off simultaneously, thereby reducing washing steps. Again, the step of contacting the first antibody with a target antigen that the first antibody specifically binds, or with a sample suspected to comprise said target antigen should be conducted prior to contacting the first antibody with the second, preferably non-monovalent, antibody that specifically binds the first antibody.

The third monovalent antibody may preferably be or comprise a single domain antibody. It may typically be an antibody from a camelid or a shark, with a camelid single domain antibody being preferred. The third monovalent antibody may be polyclonal or monoclonal with monoclonal being preferred. It is also possible to employ two or more different third antibodies that specifically recognize the same second, preferably non-monovalent, antibody but that do not cross-compete with each other. This can be achieved if the two or more third antibodies bind to different epitopes of the second, preferably non-monovalent, antibody. For example, if two of said third antibodies are used, up to four molecules of the two third antibodies can bind to one second, preferably non-monomeric, antibody. If both said third antibodies are labeled with the one or more of the same or a similar detectable label, four, six, eight, or even more (additional) detectable labels will be attached to one second, preferably non-monovalent, antibody, which provides stronger signal amplification as compared to methods, where only one third monovalent antibody is employed. It is understood that said two or more third monovalent antibodies are preferably monoclonal antibodies.

The first antibody may be any type of antibody, including an immunoglobulin, such as an immunoglobulin G (IgG), immunoglobulin A (IgA), immunoglobulin D (IgD), immunoglobulin E (IgE), immunoglobulin M (IgM), or immunoglobulin Y (IgY). It may be derived from any species, preferably a mammalian or avian species. Preferred species are e.g. rabbit, goat, donkey, guinea pig, rat, chicken, and mouse, cattle, hamster, sheep, and human. The first antibody can be polyclonal or monoclonal, with monoclonal being preferred. It is understood that the second, preferably non-monovalent, antibody specifically binds to an antibody of the same species as the first antibody, e.g. specifically bind to an antibody selected from the group consisting of rabbit, goat, donkey, guinea pig, rat, chicken, and mouse, cattle, hamster, sheep, and human, depending on the species of the first antibody. It is also possible that the second antibody specifically binds to the class of immunoglobulins of the first antibody, such as e.g. IgG, IgA, IgD, IgE, IgM, or IgY. Although not essential, it is also preferred that the second, preferably non-monovalent, antibody is not cross-reactive with an immunoglobulin of at least one second species, such as an immunoglobulin of another species selected from the group consisting of rabbit, goat, donkey, guinea pig, rat, chicken, and mouse, cattle, hamster, sheep, and human.

The second antibody may also be any type of antibody, including an immunoglobulin, such as an immunoglobulin G (IgG), immunoglobulin A (IgA), immunoglobulin D (IgD), immunoglobulin E (IgE), immunoglobulin M (IgM), or immunoglobulin Y (IgY). It may be derived from any species, preferably a mammalian or avian species. Preferred species are e.g. rabbit, goat, donkey, guinea pig, rat, chicken, and mouse, cattle, hamster, sheep, and human, depending on the species of the first antibody. However, it is envisioned by the present disclosure that the second, preferably non-monovalent, antibody is preferably derived from a species different than the species of the first antibody. The second antibody can be polyclonal or monoclonal. It is understood that the third monovalent antibody specifically binds to an antibody of the same species as the second antibody is derived from, e.g. specifically bind to an antibody selected from the group consisting of rabbit, goat, donkey, guinea pig, rat, chicken, and mouse, cattle, hamster, sheep, and human, depending on the species of the second antibody, but preferably does not bind to the an antibody of the species that the first antibody is derived from. It is also possible that the second antibody specifically binds to the class of immunoglobulins of the second, preferably non-monovalent, antibody, such as e.g., IgG, IgA, IgD, IgE, IgM, or IgY. Although not essential, it is also preferred that the third monovalent is not cross-reactive with an immunoglobulin of at least one second species, such as an immunoglobulin of another species selected from the group consisting of rabbit, goat, donkey, guinea pig, rat, chicken, and mouse, cattle, hamster, sheep, and human.

As an illustrative example, the first antibody may be a rabbit IgG, the second, preferably non-monovalent, antibody may be a goat IgG that specifically binds rabbit IgG, and the third monovalent antibody may be a camelid single domain antibody that specifically binds goat IgG. A second typical situation can be first antibody may be a mouse IgG, the second, preferably non-monovalent, antibody may be a donkey IgG that specifically binds mouse IgG, and the third monovalent antibody may be a camelid single domain antibody that specifically binds donkey IgG. More generally, the first antibody may be selected from the group consisting of a rabbit antibody, a mouse antibody, a guinea pig antibody, a hamster antibody, a goat antibody, and chicken antibody, the second antibody may specifically bind the first antibody and may be selected from the group consisting of a goat antibody, a sheep antibody, a donkey antibody, and a rabbit antibody, and the third monovalent antibody may be a camelid single domain antibody that specifically binds the second antibody

An exemplary third monovalent antibody according to the disclosure is a camelid single domain antibody specific for guinea pig IgG. Such a third monovalent antibody may have a CDR1 sequence of GLTFSEYV (SEQ ID NO: 01), a CDR2 sequence of VAAMNSRT (SEQ ID NO: 02), and a CDR3 sequence of AVGAYGDYAGPSHFHS (SEQ ID NO: 03). As an illustrative example, such a third monovalent antibody may comprise or consist of a VHH domain that has the amino acid sequence of

Another exemplary third monovalent antibody according to the disclosure is a camelid single domain antibody specific for chicken IgY. Such a third monovalent antibody may have a CDR1 sequence of GNISNILS (SEQ ID NO: 05), a CDR2 sequence of ITNDGNT (SEQ ID NO: 06), and a CDR3 sequence of NAARWTQPRPS (SEQ ID NO: 07). As an illustrative example, such a third monovalent antibody may comprise or consist of a VHH domain that has the amino acid sequence of

Another exemplary third monovalent antibody according to the disclosure is a camelid single domain antibody specific for mouse and/or rat IgG. Such a third monovalent antibody may have a CDR1 sequence of GRTDSLYA (SEQ ID NO: 09), a CDR2 sequence of ITWIGGAV (SEQ ID NO: 10), and a CDR3 sequence of ASTFRRIWDAEESIFDT (SEQ ID NO: 11). As an illustrative example, such a third monovalent antibody may comprise or consist of a VHH domain that has the amino acid sequence of

Another exemplary third monovalent antibody according to the disclosure is a camelid single domain antibody specific for human IgM. Such a third monovalent antibody may have a CDR1 sequence of GNSFSISN (SEQ ID NO: 13), a CDR2 sequence of ITSGGVA (SEQ ID NO: 14), and a CDR3 sequence of HCESGFPTLIAY (SEQ ID NO: 15). As an illustrative example, such a third monovalent antibody may comprise or consist of a VHH domain that has the amino acid sequence of

The present disclosure also relates to the use of such a third (monovalent) antibody as defined herein for enhancing a detectable signal of a fluorescent label or chromophore label of a second (preferably non-monovalent) antibody as defined herein that is bound or capable of binding to a first antibody as defined herein. It is understood that the third (monovalent) antibody is preferably a camelid single domain antibody having one or more fluorescent label(s), chromophore label(s), isotope label(s), or metal label(s) attached to it, with fluorescent label(s) being preferred.

The present invention also relates to the use of a monovalent antibody as defined in the claims having one or more fluorescent label(s) in the methods of the invention. It is understood that the monovalent antibody can be any monovalent antibody according to the methods of the invention.

The present invention also relates to the use of a monovalent antibody as defined in the claims having one or more fluorescent label(s) attached to it as a secondary antibody for detecting a target antigen by optical detection. The monovalent antibody can be any monovalent antibody according to the invention, where the monovalent antibody is used as a secondary antibody. It is understood that the use of the monovalent antibody can be according to any method of the invention that encompasses the use of the monovalent antibody as a secondary antibody.

The methods of the present invention may be used for detecting a target antigen in a sample, which may be a biological or a non-biological sample. The non-biological sample may be of chemical origin. Such a sample may comprise a protein or DNA molecule, which may be immobilized, e.g., on a glass surface or a similar surface. The sample may preferably be a biological sample. As used herein, "biological sample" may refer to any sample comprising a biological material. It may comprise a compound, cellular or sub-cellular component, which is associated with biological functions. Preferred biological samples are tissue samples, such as a biopsy or a tissue section, a blood sample, a PBMC sample, or a sample comprising a cultured cell. The biological sample may a living sample, such as e.g. a living cell, or a fixed sample, such as e.g. a fixed tissue slice or a fixed cell. Accordingly, the target antigen can be comprised and/or detected in any biological sample according to the invention. The target antigen may therefore be detected in a living cell, a fixed cell, or a fixed tissue sample etc., or in a sample comprising such a living cell, a fixed cell, or a fixed tissue sample.

The methods of the present invention are particularly advantageous, since they can be used for detection of a target antigen in a sub-cellular resolution. A "sub-cellular resolution" as used herein generally refers to a spatial resolution of the detection method that allows distinguishing between at least two molecules that are localized on or within the same, preferably mammalian, cell. A "sub-cellular" resolution preferably refers to a resolution that allows for distinguishing two points in space that are separated by a distance of about 20 µm, preferably of about 10 µm, preferably of about 5 µm, preferably of about 2 µm, preferably of about 1 µm, preferably of about 0.5 µm, preferably of about 0.2 µm, preferably of about 0.1 µm, preferably of about 0.05 µm, preferably of about 0.02 µm, preferably of about 0.01 µm.

The methods of the present invention can generally be any method that comprises the detection of a target antigen by optical detection. Accordingly, the methods of the invention may preferably be a microscopy method, such as a fluorescent microscopy method, such as an immunofluorescence method. The methods of the invention may also preferably be a flow cytometry or a fluorescence-activated cell sorting (FACS) method. The methods of the invention may also preferably be a Western Blot, Southern Blot or Northern Blot method. The methods of the invention may also be an immunohistochemistry method.

The present invention also relates to a complex as defined in appended claim 9, that can be used or formed according to the methods of the invention.

Accordingly, the present invention also relates to a complex comprising a first antibody and at least one second antibody, wherein the at least one second antibody is bound to the first antibody via the antigen binding site of the second antibody, wherein the second antibody is a monovalent antibody as defined in the claims having one or more fluorescent label(s) attached to it. It is understood that the first antibody and the second (monovalent) antibody can be any first and second (monovalent) antibodies used in the methods of the invention.

The present disclosure also relates to a complex comprising a second (preferably non-monovalent) antibody, wherein the second antibody capable of binding a first antibody via an antigen binding site of the second antibody; and at least one third antibody, wherein the at least one third antibody is bound to the second antibody via the antigen binding site of the third antibody, wherein the third antibody is a monovalent antibody having one or more fluorescent label(s), chromophore label(s), isotope label(s), or metal label(s) attached to it. The complex may further comprise the first antibody, wherein the second antibody is bound to the first antibody via an antigen binding site of the second antibody. It is understood that the first antibody, the second (preferably non-monovalent) antibody, and the third (monovalent) antibody can be any first, second (preferably non-monovalent), and third (monovalent) antibodies according to the methods of the disclosure.

The present disclosure also relates to kits that comprise components for conducting the method of the invention and/or forming the complexes of the invention

Accordingly, the present disclosure also relates to a kit comprising a first antibody; and at least one second antibody, wherein the at least one second antibody is capable of specifically binding to the first antibody via the antigen binding site of the second antibody, wherein the second antibody is a monovalent antibody having one or more fluorescent label(s), chromophore label(s), isotope label(s), or metal label(s) attached to it. It is understood that the first antibody and the second (monovalent) antibody can be any first and second (monovalent) antibodies according to the methods of the invention. In the kit, the second monovalent antibody can already be bound to the first antibody via the antigen binding site of the second antibody, such that the complex of first and (monovalent) second antibody can be directly contacted with a target antigen or the sample comprising said target antigen. In such a case, it is envisioned that two second (monovalent) antibodies are preferably bound to one first antibody. The kit may also comprise two or more different second monovalent antibodies that recognize different epitopes of the first antibody. Generally, it is envisioned that the kit comprises the second monovalent antibody and the first antibody in a molar ratio of about 1:1 to about 10:1, such as about 1.2:1 to about 8:1, preferably about 1.5:1 to about 5:1, preferably about 1.7:1 to about 3:1. It is understood that the molar ratios given herein preferably applies to each second monovalent antibody comprised in the kit.

The present disclosure also relates to a kit comprising a second (preferably non-monovalent) antibody, wherein the second (preferably non-monovalent) antibody is capable of specifically binding to a first antibody via an antigen binding site of the second antibody; and a third antibody, wherein the third antibody is capable of binding to the second antibody via the antigen binding site of the third antibody, wherein the third antibody is a monovalent antibody having one or more fluorescent label(s), chromophore label(s), isotope label(s), or metal label(s) attached to it. The kit may also comprise a first antibody. It is understood that the first antibody, the second (preferably non-monovalent) antibody, and the third (monovalent) antibody can be any first, second (preferably non-monovalent), and third (monovalent) antibodies according to the methods of the disclosure. In the kit, the third monovalent antibody can already be bound to the second (preferably non-monovalent) antibody via the antigen binding site of the third antibody, such that the complex of first and (monovalent) second antibody can be directly contacted with a first antibody that is bound to a target antigen or the sample comprising said first antibody bound to the target antigen. In such a case, it is envisioned that two third (monovalent) antibodies are preferably bound to one second (preferably non-monovalent) antibody. The kit may also comprise two or more different third monovalent antibodies that recognize different epitopes of the second antibody, or that recognize one or more epitopes on the second antibody and one or more epitopes on the first antibody. Generally, it is envisioned that the kit comprises the third monovalent antibody and the second and/or first (non-monovalent) antibody in a molar ratio of about 1:1 to about 10:1, such as about 1.2:1 to about 8:1, preferably about 1.5:1 to about 5:1, preferably about 1.7:1 to about 3:1. It is understood that the molar ratios given herein preferably applies to each pair of third monovalent antibody and first and/or second antibody comprised in the kit.

The present disclosure also relates to a camelid single domain antibody that is useful for conducting the methods of the invention. The present disclosure thus also relates to a camelid single domain antibody that specifically binds guinea pig IgG and that is not cross-reactive to mouse IgG, rat IgG, chicken IgY, rabbit IgG, human IgG, human IgM, goat IgG, donkey IgG, pig IgG, horse IgG, or cattle IgG. Such a camelid single domain antibody preferably has a CDR1 sequence of GLTFSEYV (SEQ ID NO: 01), a CDR2 sequence of VAAMNSRT (SEQ ID NO: 02), and a CDR3 sequence of AVGAYGDYAGPSHFHS (SEQ ID NO: 03). As an illustrative example, such a camelid single domain antibody may comprise or consist of a VHH domain that has the amino acid sequence of VQLLESGGGLVQAGGSLTLSCEASGLTFSEYVMGWFRQTPGKEREFVAAVAAMNSR TYYTDSVKGRFTISRDNAKNTVFLQMNSLKPEDTAVYYCAVGAYGDYAGPSHFHSW GQGTQVTVS (SEQ ID NO: 04). The present disclosure thus also relates to a camelid single domain antibody that specifically binds chicken IgY and that is not cross-reactive to guinea pig IgG, mouse IgG, rat IgG, rabbit IgG, human IgG, human IgM, goat IgG, donkey IgG, pig IgG, horse IgG, or cattle IgG. Such a camelid single domain antibody preferably has a CDR1 sequence of GNISNILS (SEQ ID NO: 05), a CDR2 sequence of ITNDGNT (SEQ ID NO: 06), and a CDR3 sequence of NAARWTQPRPS (SEQ ID NO: 07). As an illustrative example, such a camelid single domain antibody may comprise or consist of a VHH domain that has the amino acid sequence of VQLEESGGGLVEPGGSLRLSCAASGNISNILSMGWFRQAPGKRRELVATITNDGNTEY RDSVKDRFTISRDNAEKTYYLQMDRLKLEDTAVYYCNAARWTQPRPSWGQGTQVT VS (SEQ ID NO: 08). The present disclosure thus also relates to a camelid single domain antibody that specifically binds mouse or rat IgG and that is not cross-reactive to guinea pig IgG, chicken IgY, rabbit IgG, human IgG, human IgM, goat IgG, donkey IgG, pig IgG, horse IgG, or cattle IgG. Such a camelid single domain antibody preferably has a CDR1 sequence of GRTDSLYA (SEQ ID NO: 09), a CDR2 sequence of ITWIGGAV (SEQ ID NO: 10), and a CDR3 sequence of ASTFRRIWDAEESIFDT (SEQ ID NO: 11). As an illustrative example, such a camelid single domain antibody may comprise or consist of a VHH domain that has the amino acid sequence of VQLLESGGGLVQAGDSLRLSCAASGRTDSLYAVGWVRQAPGKDREFVAAITWIGGA VLYADSVKGRFTISKKFGGNTVYLQMNNLAPEDTAVYSCASTFRRIWDAEESIFDTW GQGTQVTVS (SEQ ID NO: 12). Further, the present disclosure relates to a camelid single domain antibody that specifically binds human IgM and that is not cross-reactive to guinea pig IgG, mouse IgG, rat IgG, chicken IgY, rabbit IgG, goat IgG, donkey IgG, pig IgG, horse IgG, or cattle IgG. Such a camelid single domain antibody preferably has a CDR1 sequence of GNSFSISN (SEQ ID NO: 13), a CDR2 sequence of ITSGGVA (SEQ ID NO: 14), and a CDR3 sequence of HCESGFPTLIAY (SEQ ID NO: 15). As an illustrative example, such a camelid single domain antibody may comprise or consist of a VHH domain that has the amino acid sequence of VQLLESGGGLVQFGGSLRLSCAASGNSFSISNMGWYHQAPGKERELVAIITSGGVAE YAASVKGRFTISRDYAKNTVYLQMNSLQPEDTAVYYCHCESGFPTLIAYWGQGTQV TVS (SEQ ID NO: 16). The camelid single domain antibody that specifically binds human IgM may preferably be not cross-reactive to human IgG. Further, the present disclosure relates to a camelid single domain antibody that specifically binds rabbit IgG and that is not cross-reactive to guinea pig IgG, mouse IgG, rat IgG, chicken IgY, human IgG, human IgM, goat IgG, donkey IgG, pig IgG, horse IgG, or cattle IgG. Further, the present disclosure relates to a camelid single domain antibody that specifically binds goat IgG and that is not cross-reactive to guinea pig IgG, mouse IgG, rat IgG, human IgG, human IgM, chicken IgY, rabbit IgG, donkey IgG, pig IgG, horse IgG, or cattle IgG. Further, the present disclosure relates to a camelid single domain antibody that specifically binds rat IgG and that is not cross-reactive to guinea pig IgG, mouse IgG, chicken IgY, rabbit IgG, human IgG, human IgM, goat IgG, donkey IgG, pig IgG, horse IgG, or cattle IgG. Further, the present disclosure relates to a camelid single domain antibody that specifically binds mouse IgG and that is not cross-reactive to guinea pig IgG, rat IgG, chicken IgY, rabbit IgG, human IgG, human IgM, goat IgG, donkey IgG, pig IgG, horse IgG, or cattle IgG. Further, the present disclosure relates to a camelid single domain antibody that specifically binds human IgG and that is not cross-reactive to guinea pig IgG, mouse IgG, rat IgG, chicken IgY, rabbit IgG, human IgM, goat IgG, donkey IgG, pig IgG, horse IgG, or cattle IgG. Further, the present disclosure relates to a camelid single domain antibody that specifically binds donkey IgG and that is not cross-reactive to guinea pig IgG, mouse IgG, rat IgG, chicken IgY, rabbit IgG, human IgG, human IgM, goat IgG, pig IgG, horse IgG, or cattle IgG. Further, the present disclosure relates to a camelid single domain antibody that specifically binds sheep IgG and that is not cross-reactive to guinea pig IgG, mouse IgG, rat IgG, chicken IgY, rabbit IgG, human IgG, human IgM, goat IgG, donkey IgG, pig IgG, horse IgG, or cattle IgG. It is understood that the camelid single domain antibodies of the disclosure are preferably conjugated to a fluorescent label.

The present disclosure further relates to the use of a monovalent antibody having a fluorescent label or chromophore label attached to it for detection of a molecule on the surface of a cell, wherein the use comprises contacting the monovalent antibody with the cell and detecting the fluorescent label or chromophore label. The molecule on the surface on a cell can by any molecule, such as a protein, peptide or saccharide. The molecule may be a cell surface protein or receptor. Preferably, the molecule on the surface of a cell is an immunoglobulin or a B cell receptor, preferably IgA, IgG, IgM, IgD, IgE, or IgY, preferably IgM. The cell can be any cell, preferably a mammalian or avian cell, preferably a B cell. The B cell may be a human, guinea pig, mouse, rat, chicken, rabbit, goat, donkey, pig, horse, or cattle B cell, preferably a human B cell. The fluorescent label or chromophore label can be detected by any method that is suitable for detecting such a label, including for example microscopy, immunofluorescence, flow cytometry, Western Blot, immunohistochemistry, Southern Blot, Northern blot or ELISA. The method is preferably a flow cytometry method.

The present disclosure further relates to the use of a second monovalent antibody as defined herein for improving spatial resolution and/or accuracy of a microscopy method for detecting a target antigen. The method comprises contacting a first antibody as defined herein with the target antigen and contacting the monovalent antibody with the first antibody. The improved spatial resolution and/or accuracy, which may be expressed as estimated error due to special displacement of the fluorophore, is preferably about 10 nm to about 20 nm.

The invention illustratively described herein may suitably be practiced in the absence of any element or elements, limitation or limitations, not specifically disclosed herein, as long as it falls within the scope of the appended claims. Thus for example, the terms "comprising", "including," containing", etc. shall be read expansively and without limitation.

The term "about" or "approximately" as used herein means within a deviation of 20%, such as within a deviation of 10% or within 5% of a given value or range.

### EXAMPLES

### Expression of sdAbs in Escherichia coli

DNA sequences encoding the respective sdAb were cloned into appropriate prokaryotic expression vectors in frame with N-terminal or N- and C-terminal elements encoded by the respective vector. Typically, elements N-terminally preceding the sdAb encoding region contain a PolyHis-tag C-terminal tags may be common epitope or affinity tags, fluorescent proteins, or a combination thereof. If fluorescent labeling via maleimide chemistry is desired, ectopic cysteines may be introduced at appropriate positions (Described in detail in Pleiner et al. eLife 2015;4:e11349. DOI: 10.7554/eLife.11349). In a preferred example, the expression vector contains a Tac promoter (T5 promoter fused to a Lac operator sequence) controlling the expression of the sdAb fusion and carries a Kanamycin resistance cassette as well as a Lac repressor element.

Appropriate *Escherichia coli* expression strains carrying such prokaryotic expression vectors were typically shaken over night at 30 °C in 10 ml of terrific broth (TB medium) containing appropriate antibiotics. Cultures were diluted with 50 ml fresh medium and further cultivated at 30 °C for 4-6 hours. Cultures were further diluted to 400 ml final volume with fresh medium containing 0.3 mM IPTG and shaken over night at 25 °C. Cells were harvested by centrifugation, resuspended in lysis buffer (300 mM NaCl, 50 mM Tris-HCl pH 7.5, 5 mM EDTA, 2 mM DTT) and lysed by sonication. SdAb fusions were purified from the soluble lysates obtained after removal of cell debris by centrifugation by Ni²⁺-chelate chromatography.

For attachment of fluorescent labels to accessible cysteines, the proteins were treated with 10 mM TCEP for 30 min and the buffer was exchanged to 10 mM Potassium phosphate pH 6.0, 200 mM NaCl by gel filtration. The protein-containing fractions were neutralized 1/10 volume 1 M Tris-HCl pH 8.5 and incubated with a 1.5-2-fold molar excess (relative to the number of accessible cysteines on the protein surface) of the respective maleimide-activated fluorophore for 90 min. Excess of non-reacted label was removed using appropriate chromatographic separation techniques (e.g. ion exchange chromatography, hydrophobic chromatography or size exclusion chromatography).

### Cell culture

Human Burkitt's lymphoma cells (RAMOS) were cultured using RPMI medium supplemented with 10% fetal calf serum (PAA laboratories GmbH), 4 mM glutamine and 100 U/ml of each penicillin and streptomycin (Sigma-Aldrich). Naturally occurring mutants lacking the B-cell receptor IgM (IgM(-)) were previously selected after several rounds of cell sorting (FACS).

CV-1 cells derived from African green monkey's kidney (known as COS-7 cells) and the mouse fibroblast 3T3 were grown on high glucose (4.5 g/L) Dulbecco s modified Eagle's medium (Thermo-Fischer Scientific) supplemented with 10% fetal calf serum (PAA laboratories GmbH), 4 mM glutamine and 100 U/ml of each penicillin and streptomycin (Sigma-Aldrich). Cells were seeded on poly-L-lysin coated coverslips the day before the transfection. Transfection of foreign plasmid encoding for rat Syntaxinla fused to EGFP into COS-7 cells was performed using Lipofectamin 2000 and following the instruction of the manufacturer (Thermo-Fischer Scientific).

Hippocampal primary cultures: Brains of newborn rats were removed and placed in cold HBSS (Thermo-Fischer Scientific). Hippocampi were detached from cortex and incubated 1 h in an enzyme solution (DMEM (Thermo-Fischer Scientific), 2 mg cysteine, 100 mM CaC12, 50 mM ethylenediaminetetraacetic acid (EDTA), 25 units papain per mL of solution, with CO2 bubbling, at 37°C). The enzymatic solution was removed and replaced for 5 minutes with an inactivating solution (in DMEM supplemented with 5% fetal calf serum, 25 mg albumin and 25 mg trypsin inhibitor). Hippocampi were triturated using a Pasteur pipette in Neurobasal A medium (Thermo-Fischer Scientific) supplemented with 20% B27 (Thermo-Fischer Scientific) and 10% Glutamax-I (Thermo-Fischer Scientific), 20 units/ml of penicillin and 20 µg/ml streptomycin (Roche-Diagnostics)]. Neurons were plated on 18 mm coverslips with a grown layer of rat brain astrocytes (for further details please refer to Rosenmund C, Stevens CF (1997) J Neurosci Methods).

### Immunofluorescences

Conventional immunostaining of primary hippocampal neurons (Figure 8A) was performed as follows: Cells were chemically fixed in DPBS (Dulbecco's Phosphate Buffer Saline, Sigma-Aldrich) supplemented with 4% PFA for 30 minutes. To quench remaining aldehydes, cells were briefly rinsed using DPBS and incubated for 15 minutes in DPBS containing 0.1 M glycine. The permeabilization of cell membranes and blocking of unspecific binding were achieved simultaneously by incubating the cells in BP-buffer (DPBS containing 0.1% triton X-100 and 2% bovine serum albumin) for 30 minutes at room temperature. Primary chicken polyclonal antibody against β3-tubulin (Synaptic Systems GmbH; #302306) was incubated at 1:100 dilution in BP-buffer for 60 minutes at room temperature. Three washing steps of 10 minutes each using DPBS were performed to remove excess of primary antibody. Secondary incubation was performed for 45 minutes with a 1:500 dilution in BP-buffer of a donkey anti-chicken antibody conjugated to Cy5 (Dianova GmbH; #703-175-155). Three washing steps of 10 minutes each using DPBS were performed to remove excess of antibody. Finally, cell nuclei were briefly stained using 100 nM DAPI (Thermo-Fisher Scientific), rinsed in DPBS and mounted in Mowiol (6 g glycerol; Merck Millipore, 6 ml deionized water, 12 ml 0.2 M Tris buffer pH 8.5, Merck Millipore, 2.4 g Mowiol 4-88; Merck Millipore). For Figure 8B the protocol was performed similarly. Here, however, the primary anti-β3-Tubulin antibody (1:100 dilution from stock) and secondary anti-chicken antibody coupled to Cy5 (1:500 from stock) were pre-mixed for 15 minutes in the dark and at room temperature before incubating them for 60 minutes with fixed, permeabilized and blocked hippocampal primary cultures. In Figure 8C, primary antibody (1:100 dilution from stock) was pre-mixed with 50 nM of sdAb conjugated to Cy5 for 15 minutes at room temperature and subsequently incubated for 60 minutes with the cells. Immunofluorescence of COS-7 cells in Figure 9 was performed in a similar fashion as for hippocampal neurons. However, different set of antibodies and sdAbs were used. A guinea pig anti-GFP antibody (Synaptic Systems GmbH; #132005) was used as a primary antibody (1:100 from the stock). A donkey anti-guinea pig antibody conjugated to Cy5 was used in a 1:500 dilution from stock as conventional secondary antibody (Dianova GmbH; #706-175-148). SdAb anti-guinea pig antibody coupled to Cy5 was used at 50 nM.

For Figure 10, 3T3 cells were grown on poly-L-lysine treated coverslips as described above. Permeabilized and PFA-fixed cells were treated with the indicated antibodies at the following concentrations: primary antibodies (Synaptic Systems as specified in the figure description) and secondary goat anti-mouse IgG (Jackson ImmunoResearch): 2µg/ml final concentration; fluorophore-conjugated sdAbs: 5nM each.

Live immunostainings of wild type (WT) and IgM(-) RAMOS cells were performed on 200x10⁶ cells incubated for 30 minutes on ice with DPBS and 50 nM of anti-IgM sdAb coupled to Atto647N. After 3x 10 minutes washing steps with DPBS, cells were mounted in Mowiol and imaged.

### Microscopy

Cells were imaged with an inverted epifluorescence microscope (Eclipse Ti-E; Nikon) equipped with an HBO-100W light source and a IXON X3897 Andor Technology camera. A CFI S Plan Fluor ELWD (Extra Long Working Distance; air) 20X, 0.45 NA objective (Nikon) was used for Figures 7 & 8, and a CFI Plan Apo Lambda 60X oil with 1.4 NA for Figure 6. Microscope was operated with the software NIS-Elements AR (version 4.20; Nikon).

### Cell-cytometry

Two hundred million (200x106) RAMOS cells were harvested and spun down for 60 s at 300 rpm in a tabletop centrifuge. After removing all culture medium by two washing step with cold DPBS, cells were incubated in DPBS with 1% bovine serum albumin and 5 nM of Atto647N-labeled sdAbs anti human IgM for 30 minutes on ice and dark conditions. Followed by 3 washing steps in DPBS to remove excess of unbound sdAbs. Cells were finally resuspended in cold DPBS and passed through a FACSAria II (BD Biosciences). Gated cells were further analyzed by their fluorescence intensity.

### Dot blots

To assay binding of sdAb to guinea pig, rabbit, mouse, rat, goat, bovine and human IgGs as well as chicken IgYs, the respective immunoglobins were affinity purified from serum using Protein A/G affinity columns or PEG precipitation using established protocols (Polson et al. 1980 Immunol Commun. 1980;9(5):475-93.), respectively. 0.5 µg of affinity purified IgGs were spotted onto a nitrocellulose membrane (Amersham). All further incubation steps were carried out at room temperature with shaking at 60 rpm. Dried membranes were blocked with WB buffer (5 % milk powder in Tris-buffered saline containing 0.05% Tween-20) for 30 min and washed three times for 3 min each using the same buffer. SdAbs recombinantly expressed in Escherichia coli as fusions harboring a 3x FLAG tag C-terminal of the respective sdAb. SdAb were diluted to 5 nM in WB buffer and incubated with the membranes for 60 min. The membranes were washed as before and incubated with a mouse-anti-FLAG-HRP (horseradish peroxidase) antibody (Sigma-Aldrich, clone M2) for 60 min. After washing as before, the membranes were imaged using fluorescence reader (Fujifilm LAS-4000 Mini) and the Perkin Elmer ECL-Plus kit.

### Example 1: sdAbs are able to recognize specific immunoglobulins.

Fig. 4, upper panel: Identical amounts of a MAP2 protein fragment were spotted on two nitrocellulose membranes. The left membrane was incubated with a guinea pig antibody specifically recognizing the MAP2 protein fragment (+) while the right membrane was left untreated (-). After excessive washing, both membranes were incubated with a Cy5-labeled sdAb specifically recognizing guinea pig IgGs (SEQ ID NO: 17). Fluorescence signals (black) were acquired using a fluorescence reader (Fujifilm LAS-4000 Mini). Lower panel: An analogous experiment performed using an anti-MAP2 primary antibody raised in chicken and a Cy5-labeled sdAb directed against chicken IgY (SEQ ID NO: 18).

### Example 2: Species-specific binding of sdAbs.

Native immunoglobulins (IgGs if not stated differently) from various species were spotted on a nitrocellulose membrane (∼0.5 µg), followed by the incubation with an sdAb bearing a 3xFLAG-tag and a GFP derivative on it C-terminus (SEQ ID NO: 19) (Figure 5). Chemoluminescent detection was performed using an HRP conjugated anti-FLAG-tag antibody (clone M2, **F3165,** Sigma-Aldrich), developed with ECL-Plus Kit (NEL104001EA, PerkinElmer) and imaged using a Fujifilm LAS-4000 Mini. Note that the sdAb selectively recognized only the mouse and rat IgGs. The experiment was further carried out with an anti-guinea pig sdAb bearing a 3xFLAG-tag and a GFP derivative on it C-terminus (Figure 11). Note that the sdAb selectively recognized guinea pig IgG.

### Example 3: Testing an anti-human IgM sdAb.

Fig. 6A: The specificity test shows an sdAb with selectivity towards human IgM and to some degree human IgGs (SEQ ID NO: 20). Fig. 6B: The human B-lymphocyte RAMOS cell line was stained using Cy5 coupled sdAb anti-human IgM and detected using a cell sorter (FACS). The experiment allowed to separate RAMOS cell that do not express IgMs on their surface (IgM(-); population I) from RAMOS cells expressing IgM on their surface (population II). Wild type (WT) and IgM(-) RAMOS cells were live-stained with sdAbs coupled with Cy5 and imaged under an epifluorescence microscope. Both images were acquired under the same conditions and are displayed equally scaled for direct comparison. The bright field insert image on the bottom right corner of the IgM(-) image shows the presence of cells in the field of view. Note that the use of sdAbs allow the separation of these cells without inducing an "activation" signaling cascade due to clustering IgMs (like would happen if using conventional immunoglobulins).

### Example 4: Enhancing the signal of a fluorescently labeled immunoglobulin.

Fig. 7A: 500 µg each of parvalbumin was immobilized in two separate spots on a nitrocellulose membrane and incubated with a chicken antibody directly coupled to the fluorophore Atto647N. The left spot was detected without further incubation while the protein spotted on the right side was further incubated with an anti-chicken sdAbs (SEQ ID NO: 18) coupled to Cy5. Graphs show intensity line scans along the horizontal axis together with schematic illustrations of the detected complexes. Fig. 7B: Detection of AiF using a similar setup as in A. Here, AiF was detected by either an Atto647N-labeled antibody raised in guinea pig alone (left spot) or after boosting/enhancing the signal with a Cy5-labeled sdAb directed against guinea pig IgGs (SEQ ID NO:17). Note that Atto647N and Cy5 are different molecules, but both have an equivalent excitation/emission spectrum.

### Example 5: sdAbs allow the pre-mixing of reagent and shorten significantly the time of stainings.

**Fig 8A****:** Top: Scheme depicting the basic steps in a conventional indirect immunofluorescence (IF) staining. (I) Incubation of target with primary antibody (1. Ab); typically between 0.5 and 24 h. (II) Washing the excess of 1. Ab. (III) Incubation of fluorescently labeled secondary antibody (2. Ab); typically between 0.5 and 18 h. Finally, wash off excess of 2. Ab and imaging. Middle: Example of a primary rat hippocampal neurons stained using a chicken antibody against the endogenous neuronal target β3-Tubulin (#302306 from Synaptic Systems GmbH, Göttingen Germany), detected with a conventional polyclonal anti-chicken 2. Ab coupled to Cy5 (#111-175-144 from Dianova GmbH, Hamburg Germany). Bottom: Nuclei of all (neuronal and glia) cells present in the field of view. **(B)** Ideally, to save time, the experimenter would pre-mix the 1. Ab with the 2. Ab to perform a single incubation and washing step before imaging. However, as displayed in the first image, the staining is not successful due to the clustering of 1. Ab and 2. Ab as suggested in the scheme. **(C)** Pre-mixing of a 1. Ab with a fluorescent monovalent secondary sdAb (SEQ ID NO:18) does not impair the specificity or quality of the staining. This will shorten the procedure for the immunostaining of cells and will avoid the potential clustering effect that can occur during a conventional indirect IF. This effect is illustrated in more detail in Figures 2 and 3.

### Example 6: Immunofluorescence with primary antibody premixed with secondary conventional or monovalent antibodies.

**Fig. 9A****:** A guinea pig antibody (GP) anti-EGFP was pre-mixed with a donkey anti-GP antibody coupled to Cy5 (#706-175-148 from Dianova GmbH, Hamburg Germany) and then incubated with PFA-fixed COS-7 cells transiently expressing Syntaxin1-EGFP. No antibody signal (Cy5) was visible in EGFP positive cells. Nuclear DAPI staining confirms the presence of several cells in the field of view. **(B)** Pre-mixing of the GP anti-EGFP antibody with Cy5-labeled anti-GP IgG sdAbs (SEQ ID NO: 17) resulted in a clear antibody signal (Cy5) specifically on an EGFP expressing cells, but not in the EGFP-negative cells. The DAPI nuclear staining confirms that not-transfected cells were present in the imaged field of view.

### Example 7: Multiplexing immunofluorescence with several primary mouse monoclonal antibodies, each premixed with fluorescently-labeled secondary monovalent antibodies

Multiplexing by was performed by premixing in separate tubes different mouse monoclonal antibodies (mAb) with monovalent antibodies coupled to different fluorophores for 1 hour. All pre-mixtures are pooled together and use for immunostaining the sample for 1 hour. After 3 washing steps sample can be mounted and its fluorescence measured (e.g. in microscopy, FACS, Western Blots, etc). A schematic representation of the method is shown in Figure 12 A.

Here, we show a fluorescence microscopy example of multiplexing 3 mouse monoclonal primary antibodies. After pre-mixing in separate tubes i) a primary anti tubulin antibody (Cat.No. 302-211 from SySy GmbH) with a sdAb anti-mouse IgG coupled to Atto488, ii) a primary beta-Actin (Cat.No. 251-011 from SySy GmbH) with a sdAb anti-mouse IgG coupled to Atto542, and iii) an anti-zyxin (Cat.No. 307-011 from SySy GmbH) with a sdAb-coupled to AbberiorStar635P and incubation for 1h, the mixtures were simultaneously applied to the same sample, resulting in the expected specific staining pattern for each primary antibody used. This type of multiplexing was achieved by pre-mixing primary and secondary antibodies, which is only possible if monovalent secondary antibodies are used, as essentially demonstrated in Fig 8 & 9.

## Claims

1. A method of detecting a target antigen by optical detection, comprising:
contacting a first antibody with a target antigen that said first antibody specifically binds, or with a sample suspected to comprise said target antigen
contacting the first antibody with at least one second antibody that specifically binds the first antibody, wherein the second antibody is a camelid single domain antibody having one or more fluorescent label(s) attached to it,
wherein the camelid single domain antibody is selected from the group consisting of:
(a) a camelid single domain antibody that specifically binds guinea pig IgG and that is not cross-reactive to the following antibody species: mouse IgG, rat IgG, chicken IgY, rabbit IgG, human IgG, human IgM, goat IgG, donkey IgG, pig IgG, horse IgG, and cattle IgG;
(b) a camelid single domain antibody that specifically binds chicken IgY and that is not cross-reactive to the following antibody species: guinea pig IgG, mouse IgG, rat IgG, human IgG, human IgM, goat IgG, donkey IgG, pig IgG, horse IgG, and cattle IgG;
(c) a camelid single domain antibody that specifically binds mouse or rat IgG and that is not cross-reactive to the following antibody species: guinea pig IgG, chicken IgY, rabbit IgG, human IgG, human IgM, goat IgG, donkey IgG, pig IgG, horse IgG, and cattle IgG;
(d) a camelid single domain antibody that specifically binds human IgM and that is not cross-reactive to the following antibody species: guinea pig IgG, mouse IgG, rat IgG, chicken IgY, rabbit IgG, goat IgG, donkey IgG, pig IgG, horse IgG, and cattle IgG;
(e) a camelid single domain antibody that specifically binds rabbit IgG and that is not cross-reactive to the following antibody species: guinea pig IgG, mouse IgG, rat IgG, chicken IgY, human IgG, human IgM, goat IgG, donkey IgG, pig IgG, horse IgG, and cattle IgG;
(f) a camelid single domain antibody that specifically binds goat IgG and that is not cross-reactive to the following antibody species: guinea pig IgG, mouse IgG, rat IgG, human IgG, human IgM, chicken IgY, rabbit IgG, donkey IgG, pig IgG, horse IgG, and cattle IgG;
(g) a camelid single domain antibody that specifically binds rat IgG and that is not cross-reactive to the following antibody species: guinea pig IgG, mouse IgG, chicken IgY, rabbit IgG, human IgG, human IgM, goat IgG, donkey IgG, pig IgG, horse IgG, and cattle IgG;
(h) a camelid single domain antibody that specifically binds mouse IgG and that is not cross-reactive to the following antibody species: guinea pig IgG, rat IgG, chicken IgY, rabbit IgG, human IgG, human IgM, goat IgG, donkey IgG, pig IgG, horse IgG, and cattle IgG;
(i) a camelid single domain antibody that specifically binds human IgG and that is not cross-reactive to the following antibody species: guinea pig IgG, mouse IgG, rat IgG, chicken IgY, rabbit IgG, human IgM, goat IgG, donkey IgG, pig IgG, horse IgG, and cattle IgG;
(j) a camelid single domain antibody that specifically binds donkey IgG and that is not cross-reactive to the following antibody species: guinea pig IgG, mouse IgG, rat IgG, chicken IgY, rabbit IgG, human IgG, human IgM, goat IgG, pig IgG, horse IgG, and cattle IgG; and
(k) a camelid single domain antibody that specifically binds sheep IgG and that is not cross-reactive to the following antibody species: guinea pig IgG, mouse IgG, rat IgG, chicken IgY, rabbit IgG, human IgG, human IgM, goat IgG, donkey IgG, pig IgG, horse IgG, and cattle IgG.

2. The method of claim 1, wherein the contacting of the at least one second camelid single domain antibody with the first antibody is conducted prior to contacting the first antibody with the target antigen.

3. The method of claim 1, wherein the contacting of first antibody with a target antigen and the contacting of the at least one second camelid single domain antibody with the first antibody are conducted in one single step.

4. The method of any one of the preceding claims, wherein the method is for detection of a target antigen in a biological or non-biological sample, preferably a biological sample, wherein the biological sample is preferably a tissue sample, a blood sample, a PBMC sample, or a sample comprising a cultured cell.

5. The method of any one of the preceding claims, wherein
(a) the method is for detection of the target antigen in a sub-cellular resolution;
(b) the method is for quantitative detection of the target antigen; or
(c) the target antigen is detected in a living cell or a fixed cell.

6. The method of any one of the preceding claims, wherein the method is a microscopy method.

7. The method of any one of the preceding claims, wherein the method is an immunofluorescence method.

8. The method of any one of claims 1 to 5, wherein the method is an immunohistochemistry method.

9. A complex comprising
a first antibody; and
at least one second antibody, wherein the at least one second antibody is bound to the first antibody via the antigen binding site of the second antibody, wherein the second antibody is a camelid single domain antibody having one or more fluorescent label(s) attached to it,
wherein the camelid single domain antibody is selected from the group consisting of:
(a) a camelid single domain antibody that specifically binds guinea pig IgG and that is not cross-reactive to the following antibody species: mouse IgG, rat IgG, chicken IgY, rabbit IgG, human IgG, human IgM, goat IgG, donkey IgG, pig IgG, horse IgG, and cattle IgG;
(b) a camelid single domain antibody that specifically binds chicken IgY and that is not cross-reactive to the following antibody species: guinea pig IgG, mouse IgG, rat IgG, human IgG, human IgM, goat IgG, donkey IgG, pig IgG, horse IgG, and cattle IgG;
(c) a camelid single domain antibody that specifically binds mouse or rat IgG and that is not cross-reactive to the following antibody species: guinea pig IgG, chicken IgY, rabbit IgG, human IgG, human IgM, goat IgG, donkey IgG, pig IgG, horse IgG, and cattle IgG;
(d) a camelid single domain antibody that specifically binds human IgM and that is not cross-reactive to the following antibody species: guinea pig IgG, mouse IgG, rat IgG, chicken IgY, rabbit IgG, goat IgG, donkey IgG, pig IgG, horse IgG, and cattle IgG;
(e) a camelid single domain antibody that specifically binds rabbit IgG and that is not cross-reactive to the following antibody species: guinea pig IgG, mouse IgG, rat IgG, chicken IgY, human IgG, human IgM, goat IgG, donkey IgG, pig IgG, horse IgG, and cattle IgG;
(f) a camelid single domain antibody that specifically binds goat IgG and that is not cross-reactive to the following antibody species: guinea pig IgG, mouse IgG, rat IgG, human IgG, human IgM, chicken IgY, rabbit IgG, donkey IgG, pig IgG, horse IgG, and cattle IgG;
(g) a camelid single domain antibody that specifically binds rat IgG and that is not cross-reactive to the following antibody species: guinea pig IgG, mouse IgG, chicken IgY, rabbit IgG, human IgG, human IgM, goat IgG, donkey IgG, pig IgG, horse IgG, and cattle IgG;
(h) a camelid single domain antibody that specifically binds mouse IgG and that is not cross-reactive to the following antibody species: guinea pig IgG, rat IgG, chicken IgY, rabbit IgG, human IgG, human IgM, goat IgG, donkey IgG, pig IgG, horse IgG, and cattle IgG;
(i) a camelid single domain antibody that specifically binds human IgG and that is not cross-reactive to the following antibody species: guinea pig IgG, mouse IgG, rat IgG, chicken IgY, rabbit IgG, human IgM, goat IgG, donkey IgG, pig IgG, horse IgG, and cattle IgG;
(j) a camelid single domain antibody that specifically binds donkey IgG and that is not cross-reactive to the following antibody species: guinea pig IgG, mouse IgG, rat IgG, chicken IgY, rabbit IgG, human IgG, human IgM, goat IgG, pig IgG, horse IgG, and cattle IgG;
(k) a camelid single domain antibody that specifically binds sheep IgG and that is not cross-reactive to the following antibody species: guinea pig IgG, mouse IgG, rat IgG, chicken IgY, rabbit IgG, human IgG, human IgM, goat IgG, donkey IgG, pig IgG, horse IgG, and cattle IgG.

10. The complex of claim 9, wherein two second camelid single domain antibodies are bound to the first antibody.

11. The complex of claim 9 or 10, wherein the complex is comprised in a biological or non-biological sample, preferably a biological sample, or wherein the complex is comprised in a living cell or a fixed cell.

12. The complex of any one of claims 9 to 11, wherein the complex is not cross-linked to another first antibody.

13. Use of a camelid single domain antibody having one or more fluorescent label(s) attached to it as a secondary antibody for detecting a target antigen by optical detection, wherein the camelid single domain antibody is selected from the group consisting of:
(a) a camelid single domain antibody that specifically binds guinea pig IgG and that is not cross-reactive to the following antibody species: mouse IgG, rat IgG, chicken IgY, rabbit IgG, human IgG, human IgM, goat IgG, donkey IgG, pig IgG, horse IgG, and cattle IgG;
(b) a camelid single domain antibody that specifically binds chicken IgY and that is not cross-reactive to the following antibody species: guinea pig IgG, mouse IgG, rat IgG, human IgG, human IgM, goat IgG, donkey IgG, pig IgG, horse IgG, and cattle IgG;
(c) a camelid single domain antibody that specifically binds mouse or rat IgG and that is not cross-reactive to the following antibody species: guinea pig IgG, chicken IgY, rabbit IgG, human IgG, human IgM, goat IgG, donkey IgG, pig IgG, horse IgG, and cattle IgG;
(d) a camelid single domain antibody that specifically binds human IgM and that is not cross-reactive to the following antibody species: guinea pig IgG, mouse IgG, rat IgG, chicken IgY, rabbit IgG, goat IgG, donkey IgG, pig IgG, horse IgG, and cattle IgG;
(e) a camelid single domain antibody that specifically binds rabbit IgG and that is not cross-reactive to the following antibody species: guinea pig IgG, mouse IgG, rat IgG, chicken IgY, human IgG, human IgM, goat IgG, donkey IgG, pig IgG, horse IgG, and cattle IgG;
(f) a camelid single domain antibody that specifically binds goat IgG and that is not cross-reactive to the following antibody species: guinea pig IgG, mouse IgG, rat IgG, human IgG, human IgM, chicken IgY, rabbit IgG, donkey IgG, pig IgG, horse IgG, and cattle IgG;
(g) a camelid single domain antibody that specifically binds rat IgG and that is not cross-reactive to the following antibody species: guinea pig IgG, mouse IgG, chicken IgY, rabbit IgG, human IgG, human IgM, goat IgG, donkey IgG, pig IgG, horse IgG, and cattle IgG;
(h) a camelid single domain antibody that specifically binds mouse IgG and that is not cross-reactive to the following antibody species: guinea pig IgG, rat IgG, chicken IgY, rabbit IgG, human IgG, human IgM, goat IgG, donkey IgG, pig IgG, horse IgG, and cattle IgG;
(i) a camelid single domain antibody that specifically binds human IgG and that is not cross-reactive to the following antibody species: guinea pig IgG, mouse IgG, rat IgG, chicken IgY, rabbit IgG, human IgM, goat IgG, donkey IgG, pig IgG, horse IgG, and cattle IgG;
(j) a camelid single domain antibody that specifically binds donkey IgG and that is not cross-reactive to the following antibody species: guinea pig IgG, mouse IgG, rat IgG, chicken IgY, rabbit IgG, human IgG, human IgM, goat IgG, pig IgG, horse IgG, and cattle IgG; and
(k) a camelid single domain antibody that specifically binds sheep IgG and that is not cross-reactive to the following antibody species: guinea pig IgG, mouse IgG, rat IgG, chicken IgY, rabbit IgG, human IgG, human IgM, goat IgG, donkey IgG, pig IgG, horse IgG, and cattle IgG.

## Patentansprüche

1. Verfahren zum Nachweis eines Zielantigens durch optische Detektion, umfassend:
Inkontaktbringen eines ersten Antikörpers mit einem Zielantigen, das der erste Antikörper spezifisch bindet, oder mit einer Probe, bei der der Verdacht besteht, dass sie das Zielantigen umfasst,
Inkontaktbringen des ersten Antikörpers mit mindestens einem zweiten Antikörper, der den ersten Antikörper spezifisch bindet, wobei der zweite Antikörper ein Kamel-Single-Domain-Antikörper mit einer oder mehreren daran angebrachten Fluoreszenzmarkierungen ist,
wobei der Kamel-Single-Domain-Antikörper ausgewählt ist, aus der Gruppe bestehend aus
(a) Kamel-Single-Domain-Antikörper, der spezifisch Meerschweinchen-IgG bindet und nicht mit den folgenden Antikörperspezies kreuzreagiert: Maus-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(b) Kamel-Single-Domain-Antikörper, der spezifisch Hühner-IgY bindet und nicht mit den folgenden Antikörperspezies kreuzreagiert: Meerschweinchen-IgG, Maus-IgG, Ratten-IgG, Menschen-IgG, Menschen-IgM, Ziegen-IgG, Esel-IgG, Schweine-IgG, Pferde-IgG und Rinder-IgG;
(c) Kamel-Single-Domain-Antikörper, der spezifisch Maus- oder Ratten-IgG bindet und nicht mit den folgenden Antikörperspezies kreuzreagiert: Meerschweinchen-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(d) Kamel-Single-Domain-Antikörper, der spezifisch Menschen-IgM bindet und nicht mit den folgenden Antikörperspezies kreuzreagiert: Meerschweinchen-IgG, Maus-IgG, Ratten-IgG, Hühner-IgY, Kaninchen-IgG, Ziegen-IgG, Esel-IgG, Schweine-IgG, Pferde-IgG und Rinder-IgG;
(e) Kamel-Single-Domain-Antikörper, der spezifisch Kaninchen-IgG bindet und nicht mit den folgenden Antikörperspezies kreuzreagiert: Meerschweinchen-IgG, Maus-IgG, Ratten-IgG, Hühner-IgY, Menschen-IgG, Menschen-IgM, Ziegen-IgG, Esel-IgG, Schweine-IgG, Pferde-IgG und Rinder-IgG;
(f) Kamel-Single-Domain-Antikörper, der spezifisch Ziegen-IgG bindet und nicht mit den folgenden Antikörperspezies kreuzreagiert: Meerschweinchen-IgG, Maus-IgG, Ratten-IgG, Menschen-IgG, Menschen-IgM, Hühner-IgY, Kaninchen-IgG, Esel-IgG, Schweine-IgG, Pferde-IgG und Rinder-IgG;
(g) Kamel-Single-Domain-Antikörper, der spezifisch Ratten-IgG bindet und nicht mit den folgenden Antikörperspezies kreuzreagiert: Meerschweinchen-IgG, Maus-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(h) Kamel-Single-Domain-Antikörper, der spezifisch Maus-IgG bindet und nicht mit den folgenden Antikörperspezies kreuzreagiert: Meerschweinchen-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(i) Kamel-Single-Domain-Antikörper, der spezifisch Menschen-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgM, Ziegen-IgG, Esel-IgG, Schweine-IgG, Pferde-IgG und Rinder-IgG;
(j) Kamel-Single-Domain-Antikörper, der spezifisch Esel-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgG, Menschen-IgM, Ziegen-IgG, Schweine-IgG, Pferde-IgG und Rinder-IgG; und
(k) Kamel-Single-Domain-Antikörper, der spezifisch Schaf-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG.

2. Verfahren nach Anspruch 1, wobei das Inkontaktbringen des mindestens einen zweiten Kamel-Single-Domain-Antikörpers mit dem ersten Antikörper vor dem Inkontaktbringen des ersten Antikörpers mit dem Zielantigen durchgeführt wird.

3. Verfahren Anspruch 1, wobei das Inkontaktbringen des ersten Antikörpers mit einem Zielantigen und das Inkontaktbringen des mindestens einen zweiten Kamel-Single-Domain-Antikörpers mit dem ersten Antikörper in einem einzigen Schritt durchgeführt werden.

4. Verfahren nach einem der vorhergehenden Ansprüche, wobei das Verfahren zum Nachweis eines Zielantigens in einer biologischen oder nicht-biologischen Probe, vorzugsweise einer biologischen Probe, dient, wobei die biologische Probe vorzugsweise eine Gewebeprobe, eine Blutprobe, eine PBMC-Probe oder eine Probe ist, die eine kultivierte Zelle umfasst.

5. Verfahren nach einem der vorhergehenden Ansprüche, wobei
(a) das Verfahren zum Nachweis des Zielantigens in einer subzellulären Auflösung ist;
(b) das Verfahren zum quantitativen Nachweis des Zielantigens ist; oder
(c) das Zielantigen in einer lebenden Zelle oder einer fixierten Zelle nachgewiesen wird.

6. Verfahren nach einem der vorhergehenden Ansprüche, wobei das Verfahren ein Mikroskopieverfahren ist.

7. Verfahren nach einem der vorhergehenden Ansprüche, wobei das Verfahren ein Immunfluoreszenzverfahren ist.

8. Verfahren nach einem der Ansprüche 1 bis 5, wobei das Verfahren ein Immunhistochemieverfahren ist.

9. Komplex, umfassend
einen ersten Antikörper; und
mindestens einen zweiten Antikörper, wobei der mindestens eine zweite Antikörper über die Antigenbindungsstelle des zweiten Antikörpers an den ersten Antikörper gebunden ist, wobei der zweite Antikörper ein Kamel-Single-Domain-Antikörper mit einer oder mehreren daran angebrachten Fluoreszenzmarkierungen ist,
wobei der Kamel-Single-Domain-Antikörper ausgewählt ist, aus der Gruppe bestehend aus
(a) Kamel-Single-Domain-Antikörper, der spezifisch Meerschweinchen-IgG bindet und nicht mit den folgenden Antikörperspezies kreuzreagiert: Maus-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(b) Kamel-Single-Domain-Antikörper, der spezifisch Hühner-IgY bindet und nicht mit den folgenden Antikörperspezies kreuzreagiert: Meerschweinchen-IgG, Maus-IgG, Ratten-IgG, Menschen-IgG, Menschen-IgM, Ziegen-IgG, Esel-IgG, Schweine-IgG, Pferde-IgG und Rinder-IgG;
(c) Kamel-Single-Domain-Antikörper, der spezifisch Maus- oder Ratten-IgG bindet und nicht mit den folgenden Antikörperspezies kreuzreagiert: Meerschweinchen-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(d) Kamel-Single-Domain-Antikörper, der spezifisch Menschen-IgM bindet und nicht mit den folgenden Antikörperspezies kreuzreagiert: Meerschweinchen-IgG, Maus-IgG, Ratten-IgG, Hühner-IgY, Kaninchen-IgG, Ziegen-IgG, Esel-IgG, Schweine-IgG, Pferde-IgG und Rinder-IgG;
(e) Kamel-Single-Domain-Antikörper, der spezifisch Kaninchen-IgG bindet und nicht mit den folgenden Antikörperspezies kreuzreagiert: Meerschweinchen-IgG, Maus-IgG, Ratten-IgG, Hühner-IgY, Menschen-IgG, Menschen-IgM, Ziegen-IgG, Esel-IgG, Schweine-IgG, Pferde-IgG und Rinder-IgG;
(f) Kamel-Single-Domain-Antikörper, der spezifisch Ziegen-IgG bindet und nicht mit den folgenden Antikörperspezies kreuzreagiert: Meerschweinchen-IgG, Maus-IgG, Ratten-IgG, Menschen-IgG, Menschen-IgM, Hühner-IgY, Kaninchen-IgG, Esel-IgG, Schweine-IgG, Pferde-IgG und Rinder-IgG;
(g) Kamel-Single-Domain-Antikörper, der spezifisch Ratten-IgG bindet und nicht mit den folgenden Antikörperspezies kreuzreagiert: Meerschweinchen-IgG, Maus-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(h) Kamel-Single-Domain-Antikörper, der spezifisch Maus-IgG bindet und nicht mit den folgenden Antikörperspezies kreuzreagiert: Meerschweinchen-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(i) Kamel-Single-Domain-Antikörper, der spezifisch Menschen-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgM, Ziegen-IgG, Esel-IgG, Schweine-IgG, Pferde-IgG und Rinder-IgG;
(j) Kamel-Single-Domain-Antikörper, der spezifisch Esel-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgG, Menschen-IgM, Ziegen-IgG, Schweine-IgG, Pferde-IgG und Rinder-IgG; und
(k) Kamel-Single-Domain-Antikörper, der spezifisch Schaf-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG.

10. Komplex nach Anspruch 9, wobei zwei zweite Kamel-Single-Domain-Antikörper an den ersten Antikörper gebunden sind.

11. Komplex nach Anspruch 9 oder 10, wobei der Komplex in einer biologischen oder nicht-biologischen Probe, vorzugsweise einer biologischen Probe, enthalten ist, oder wobei der Komplex in einer lebenden Zelle oder einer fixierte Zelle enthalten ist.

12. Komplex nach einem der Ansprüche 9 bis 11, wobei der Komplex nicht mit einem anderen ersten Antikörper quervernetzt ist.

13. Verwendung eines Kamel-Single-Domain-Antikörpers mit einer oder mehreren daran angebrachten Fluoreszenzmarkierungen als sekundärer Antikörper zum Nachweis eines Zielantigens durch optischen Nachweis, wobei der Kamel-Single-Domain-Antikörper ausgewählt ist, aus der Gruppe bestehend aus
(a) Kamel-Single-Domain-Antikörper, der spezifisch Meerschweinchen-IgG bindet und nicht mit den folgenden Antikörperspezies kreuzreagiert: Maus-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(b) Kamel-Single-Domain-Antikörper, der spezifisch Hühner-IgY bindet und nicht mit den folgenden Antikörperspezies kreuzreagiert: Meerschweinchen-IgG, Maus-IgG, Ratten-IgG, Menschen-IgG, Menschen-IgM, Ziegen-IgG, Esel-IgG, Schweine-IgG, Pferde-IgG und Rinder-IgG;
(c) Kamel-Single-Domain-Antikörper, der spezifisch Maus- oder Ratten-IgG bindet und nicht mit den folgenden Antikörperspezies kreuzreagiert: Meerschweinchen-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(d) Kamel-Single-Domain-Antikörper, der spezifisch Menschen-IgM bindet und nicht mit den folgenden Antikörperspezies kreuzreagiert: Meerschweinchen-IgG, Maus-IgG, Ratten-IgG, Hühner-IgY, Kaninchen-IgG, Ziegen-IgG, Esel-IgG, Schweine-IgG, Pferde-IgG und Rinder-IgG;
(e) Kamel-Single-Domain-Antikörper, der spezifisch Kaninchen-IgG bindet und nicht mit den folgenden Antikörperspezies kreuzreagiert: Meerschweinchen-IgG, Maus-IgG, Ratten-IgG, Hühner-IgY, Menschen-IgG, Menschen-IgM, Ziegen-IgG, Esel-IgG, Schweine-IgG, Pferde-IgG und Rinder-IgG;
(f) Kamel-Single-Domain-Antikörper, der spezifisch Ziegen-IgG bindet und nicht mit den folgenden Antikörperspezies kreuzreagiert: Meerschweinchen-IgG, Maus-IgG, Ratten-IgG, Menschen-IgG, Menschen-IgM, Hühner-IgY, Kaninchen-IgG, Esel-IgG, Schweine-IgG, Pferde-IgG und Rinder-IgG;
(g) Kamel-Single-Domain-Antikörper, der spezifisch Ratten-IgG bindet und nicht mit den folgenden Antikörperspezies kreuzreagiert: Meerschweinchen-IgG, Maus-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(h) Kamel-Single-Domain-Antikörper, der spezifisch Maus-IgG bindet und nicht mit den folgenden Antikörperspezies kreuzreagiert: Meerschweinchen-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG;
(i) Kamel-Single-Domain-Antikörper, der spezifisch Menschen-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgM, Ziegen-IgG, Esel-IgG, Schweine-IgG, Pferde-IgG und Rinder-IgG;
(j) Kamel-Single-Domain-Antikörper, der spezifisch Esel-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgG, Menschen-IgM, Ziegen-IgG, Schweine-IgG, Pferde-IgG und Rinder-IgG; und
(k) Kamel-Single-Domain-Antikörper, der spezifisch Schaf-IgG bindet und nicht mit den folgenden Antikörperspezies kreuzreagiert: Meerschweinchen-IgG, Maus-IgG, Ratten-IgG, Hühner-IgY, Kaninchen-IgG, Menschen-IgG, Menschen-IgM, Ziegen-IgG, Esel-IgG, Schweine-IgG, Pferde-IgG und Rinder-IgG.

## Revendications

1. Procédé de détection d'un antigène cible par détection optique, comportant:
mise en contact d'un premier anticorps avec un antigène cible que ledit premier anticorps lie spécifiquement, ou avec un échantillon suspecté de comprendre ledit antigène cible contactant le premier anticorps avec au moins un second anticorps qui lie spécifiquement le premier anticorps, dans lequel le second anticorps est un anticorps à domaine unique de camélidés ayant un ou plusieurs marqueur(s) fluorescent(s) attaché(s) à celui-ci,
dans lequel l'anticorps à domaine unique de camélidés est choisi parmi le groupe, constitué de:
(a) un anticorps à domaine unique de camélidés liant spécifiquement l'IgG de cobaye et qui n'est pas à réaction croisée avec les espèces d'anticorps suivantes: l'IgG de souris, l' l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(b) un anticorps à domaine unique de camélidés liant spécifiquement l'IgY de poulet et qui n'est pas à réaction croisée avec les espèces d'anticorps suivantes: l'IgG de cobaye, l'IgG de souris, l'IgG de rat, l'IgG humaine, l'IgM humaine, l'IgG de chèvre, l'IgG d'âne, l'IgG de porc, l'IgG de cheval, et l'IgG de bétail;
(c) un anticorps à domaine unique de camélidés liant spécifiquement l'IgG de souris ou l'IgG de rat et qui n'est pas à réaction croisée avec les espèces d'anticorps suivantes: l'IgG de cobaye, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(d) un anticorps à domaine unique de camélidés liant spécifiquement l'IgM humaine et qui n'est pas à réaction croisée avec les espèces d'anticorps suivantes: l'IgG de cobaye, l'IgG de souris, l'IgG de rat, l'IgY de poulet, l'IgG de lapin, l'IgG de chèvre, l'IgG d'âne, l'IgG de porc, l'IgG de cheval, et l'IgG de bétail;
(e) un anticorps à domaine unique de camélidés liant spécifiquement l'IgG de lapin et qui n'est pas à réaction croisée avec les espèces d'anticorps suivantes: l'IgG de cobaye, l'IgG de souris, l'IgG de rat, l'IgY de poulet, l'IgG humaine, l'IgM humaine, l'IgG de chèvre, l'IgG d'âne, l'IgG de porc, l'IgG de cheval, et l'IgG de bétail;
(f) un anticorps à domaine unique de camélidés liant spécifiquement l'IgG de chèvre et qui n'est pas à réaction croisée avec les espèces d'anticorps suivantes: l'IgG de cobaye, l'IgG de souris, l'IgG de rat, l'IgG humaine, l'IgM humaine, l'IgY de poulet, l'IgG de lapin, l'IgG d'âne, l'IgG de porc, l'IgG de cheval, et l'IgG de bétail;
(g) un anticorps à domaine unique de camélidés liant spécifiquement l'IgG de rat et qui n'est pas à réaction croisée avec les espèces d'anticorps suivantes: l'IgG de cobaye, l'IgG de souris, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(h) un anticorps à domaine unique de camélidés liant spécifiquement l'IgG de souris et qui n'est pas à réaction croisée avec les espèces d'anticorps suivantes: l'IgG de cobaye, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(i) un anticorps à domaine unique de camélidés liant spécifiquement l'IgG humaine et qui n'est pas à réaction croisée avec les espèces d'anticorps suivantes: l'IgG de cobaye, l'IgG de souris, l'IgG de rat, l'IgY de poulet, l'IgG de lapin, l'IgM humaine, l'IgG de chèvre, l'IgG d'âne, l'IgG de porc, l'IgG de cheval, et l'IgG de bétail;
(j) un anticorps à domaine unique de camélidés liant spécifiquement l'IgG d'âne et qui n'est pas à réaction croisée avec les espèces d'anticorps suivantes: l'IgG de cobaye, l'IgG de souris, l'IgG de rat, l'IgY de poulet, l'IgG de lapin, l'IgG humaine, l'IgM humaine, l'IgG de chèvre, l'IgG de porc, l'IgG de cheval, et l'IgG de bétail;
(k) un anticorps à domaine unique de camélidés liant spécifiquement l'IgG de mouton et qui n'est pas à réaction croisée avec les espèces d'anticorps suivantes: l'IgG de cobaye, l'IgG de souris, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail.

2. Procédé selon la revendication 1, dans lequel la mise en contact de l'au moins un second anticorps à domaine unique de camélidés avec le premier anticorps est effectuée avant la mise en contact du premier anticorps avec l'antigène cible.

3. Procédé selon la revendication 1, dans lequel la mise en contact du premier anticorps avec un antigène cible et la mise en contact de l'au moins un second anticorps à domaine unique de camélidés avec le premier anticorps sont effectuées en une seule étape.

4. Procédé selon l'une quelconque des revendications précédentes, dans lequel le procédé est pour la détection d'un antigène cible dans un échantillon biologique ou non biologique, de préférence, un échantillon biologique, dans lequel l'échantillon biologique est, de préférence, un échantillon de tissu, un échantillon de sang, un échantillon PBMC ou un échantillon comportant une cellule cultivée.

5. Procédé selon l'une quelconque des revendications précédentes, dans lequel
(a) le procédé est pour la détection de l'antigène cible dans une résolution sous-cellulaire;
(b) le procédé est pour la détection quantitative de l'antigène cible; ou
(c) l'antigène cible est détecté dans une cellule vivante ou une cellule fixée.

6. Procédé selon l'une quelconque des revendications précédentes, dans lequel le procédé est un procédé de microscopie.

7. Procédé selon l'une quelconque des revendications précédentes, dans lequel le procédé est un procédé d'immunofluorescence.

8. Procédé selon l'une quelconque des revendications 1 à 5, dans lequel le procédé est un procédé d'immunohistochimie.

9. Complexe, comportant
un premier anticorps; et
au moins un second anticorps, dans lequel l'au moins un second anticorps est lié au premier anticorps via le site de liaison de l'antigène du second anticorps, dans lequel le second anticorps est un anticorps à domaine unique de camélidés ayant un ou plusieurs marqueur(s) fluorescent(s) attaché(s) à celui-ci,
dans lequel l'anticorps à domaine unique de camélidés est choisi parmi le groupe, constitué de:
(a) un anticorps à domaine unique de camélidés liant spécifiquement l'IgG de cobaye et qui n'est pas à réaction croisée avec les espèces d'anticorps suivantes: l'IgG de souris, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(b) un anticorps à domaine unique de camélidés liant spécifiquement l'IgY de poulet et qui n'est pas à réaction croisée avec les espèces d'anticorps suivantes: l'IgG de cobaye, l'IgG de souris, l'IgG de rat, l'IgG humaine, l'IgM humaine, l'IgG de chèvre, l'IgG d'âne, l'IgG de porc, l'IgG de cheval, et l'IgG de bétail;
(c) un anticorps à domaine unique de camélidés liant spécifiquement l'IgG de souris ou l'IgG de rat et qui n'est pas à réaction croisée avec les espèces d'anticorps suivantes: l'IgG de cobaye, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(d) un anticorps à domaine unique de camélidés liant spécifiquement l'IgM humaine et qui n'est pas à réaction croisée avec les espèces d'anticorps suivantes: l'IgG de cobaye, l'IgG de souris, l'IgG de rat, l'IgY de poulet, l'IgG de lapin, l'IgG de chèvre, l'IgG d'âne, l'IgG de porc, l'IgG de cheval, et l'IgG de bétail;
(e) un anticorps à domaine unique de camélidés liant spécifiquement l'IgG de lapin et qui n'est pas à réaction croisée avec les espèces d'anticorps suivantes: l'IgG de cobaye, l'IgG de souris, l'IgG de rat, l'IgY de poulet, l'IgG humaine, l'IgM humaine, l'IgG de chèvre, l'IgG d'âne, l'IgG de porc, l'IgG de cheval, et l'IgG de bétail;
(f) un anticorps à domaine unique de camélidés liant spécifiquement l'IgG de chèvre et qui n'est pas à réaction croisée avec les espèces d'anticorps suivantes: l'IgG de cobaye, l'IgG de souris, l'IgG de rat, l'IgG humaine, l'IgM humaine, l'IgY de poulet, l'IgG de lapin, l'IgG d'âne, l'IgG de porc, l'IgG de cheval, et l'IgG de bétail;
(g) un anticorps à domaine unique de camélidés liant spécifiquement l'IgG de rat et qui n'est pas à réaction croisée avec les espèces d'anticorps suivantes: l'IgG de cobaye, l'IgG de souris, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(h) un anticorps à domaine unique de camélidés liant spécifiquement l'IgG de souris et qui n'est pas à réaction croisée avec les espèces d'anticorps suivantes: l'IgG de cobaye, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(i) un anticorps à domaine unique de camélidés liant spécifiquement l'IgG humaine et qui n'est pas à réaction croisée avec les espèces d'anticorps suivantes: l'IgG de cobaye, l'IgG de souris, l'IgG de rat, l'IgY de poulet, l'IgG de lapin, l'IgM humaine, l'IgG de chèvre, l'IgG d'âne, l'IgG de porc, l'IgG de cheval, et l'IgG de bétail;
(j) un anticorps à domaine unique de camélidés liant spécifiquement l'IgG d'âne et qui n'est pas à réaction croisée avec les espèces d'anticorps suivantes: l'IgG de cobaye, l'IgG de souris, l'IgG de rat, l'IgY de poulet, l'IgG de lapin, l'IgG humaine, l'IgM humaine, l'IgG de chèvre, l'IgG de porc, l'IgG de cheval, et l'IgG de bétail;
(k) un anticorps à domaine unique de camélidés liant spécifiquement l'IgG de mouton et qui n'est pas à réaction croisée avec les espèces d'anticorps suivantes: l'IgG de cobaye, l'IgG de souris, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail.

10. Complexe selon la revendication 9, dans lequel deux seconds anticorps à domaine unique de camélidés sont liés au premier anticorps.

11. Complexe selon la revendication 9 ou 10, dans lequel le complexe est compris dans un échantillon biologique ou non biologique, de préférence, un échantillon biologique, ou dans lequel le complexe est compris dans une cellule vivante ou une cellule fixée.

12. Complexe selon l'une quelconque des revendications 9 à 11, dans lequel le complexe n'est pas réticulé à un autre premier anticorps.

13. Utilisation d'un anticorps à domaine unique de camélidés ayant un ou plusieurs marqueur(s) fluorescent(s) attaché(s) à celui-ci comme anticorps secondaire pour détecter un antigène cible par détection optique, dans lequel l'anticorps à domaine unique de camélidés est choisi parmi le groupe, constitué de:
(a) un anticorps à domaine unique de camélidés liant spécifiquement l'IgG de cobaye et qui n'est pas à réaction croisée avec les espèces d'anticorps suivantes: l'IgG de souris, l' l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(b) un anticorps à domaine unique de camélidés liant spécifiquement l'IgY de poulet et qui n'est pas à réaction croisée avec les espèces d'anticorps suivantes: l'IgG de cobaye, l'IgG de souris, l'IgG de rat, l'IgG humaine, l'IgM humaine, l'IgG de chèvre, l'IgG d'âne, l'IgG de porc, l'IgG de cheval, et l'IgG de bétail;
(c) un anticorps à domaine unique de camélidés liant spécifiquement l'IgG de souris ou de rat et qui n'est pas à réaction croisée avec les espèces d'anticorps suivantes: l'IgG de cobaye, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(d) un anticorps à domaine unique de camélidés liant spécifiquement l'IgM humaine et qui n'est pas à réaction croisée avec les espèces d'anticorps suivantes: l'IgG de cobaye, l'IgG de souris, l'IgG de rat, l'IgY de poulet, l'IgG de lapin, l'IgG de chèvre, l'IgG d'âne, l'IgG de porc, l'IgG de cheval, et l'IgG de bétail;
(e) un anticorps à domaine unique de camélidés liant spécifiquement l'IgG de lapin et qui n'est pas à réaction croisée avec les espèces d'anticorps suivantes: l'IgG de cobaye, l'IgG de souris, l'IgG de rat, l'IgY de poulet, l'IgG humaine, l'IgM humaine, l'IgG de chèvre, l'IgG d'âne, l'IgG de porc, l'IgG de cheval, et l'IgG de bétail;
(f) un anticorps à domaine unique de camélidés liant spécifiquement l'IgG de chèvre et qui n'est pas à réaction croisée avec les espèces d'anticorps suivantes: l'IgG de cobaye, l'IgG de souris, l'IgG de rat, l'IgG humaine, l'IgM humaine, l'IgY de poulet, l'IgG de lapin, l'IgG d'âne, l'IgG de porc, l'IgG de cheval, et l'IgG de bétail;
(g) un anticorps à domaine unique de camélidés liant spécifiquement l'IgG de rat et qui n'est pas à réaction croisée avec les espèces d'anticorps suivantes: l'IgG de cobaye, l'IgG de souris, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(h) un anticorps à domaine unique de camélidés liant spécifiquement l'IgG de souris et qui n'est pas à réaction croisée avec les espèces d'anticorps suivantes: l'IgG de cobaye, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail;
(i) un anticorps à domaine unique de camélidés liant spécifiquement l'IgG humaine et qui n'est pas à réaction croisée avec les espèces d'anticorps suivantes: l'IgG de cobaye, l'IgG de souris, l'IgG de rat, l'IgY de poulet, l'IgG de lapin, l'IgM humaine, l'IgG de chèvre, l'IgG d'âne, l'IgG de porc, l'IgG de cheval, et l'IgG de bétail;
(j) un anticorps à domaine unique de camélidés liant spécifiquement l'IgG d'âne et qui n'est pas à réaction croisée avec les espèces d'anticorps suivantes: l'IgG de cobaye, l'IgG de souris, l'IgG de rat, l'IgY de poulet, l'IgG de lapin, l'IgG humaine, l'IgM humaine, l'IgG de chèvre, l'IgG de porc, l'IgG de cheval, et l'IgG de bétail;
(k) un anticorps à domaine unique de camélidés liant spécifiquement l'IgG de mouton et qui n'est pas à réaction croisée avec les espèces d'anticorps suivantes: l'IgG de cobaye, l'IgG de souris, l'IgG de rat, l'IgY de poulet, l'IgG de lapin, l'IgG humaine, l'IgM humaine, l'IgG de chèvre, l'IgG d'âne, l'IgG de porc, l'IgG de cheval, et l'IgG de bétail.


### SEQUENCE LISTING

<110> Nanotag Biotechnologies GmbH
<120> TARGET DETECTION USING A MONOVALENT ANTIBODY
<130> NAN15884PCT
<150> EP 17 160 720.3
   <151> 2017-03-14
<160> 20
<170> PatentIn version 3.5
<210> 1
   <211> 8
   <212> PRT
   <213> Artificial Sequence
<220>
   <223> camelid VHH CDR1
<400> 1
   Gly Leu Thr Phe Ser Glu Tyr Val
   1               5
<210> 2
   <211> 8
   <212> PRT
   <213> Artificial Sequence
<220>
   <223> camelid VHH CDR2
<400> 2
   Val Ala Ala Met Asn Ser Arg Thr
   1               5
<210> 3
   <211> 16
   <212> PRT
   <213> Artificial Sequence
<220>
   <223> camelid VHH CDR3
<400> 3
   Ala Val Gly Ala Tyr Gly Asp Tyr Ala Gly Pro Ser His Phe His Ser
   1               5                   10                  15
<210> 4
   <211> 121
   <212> PRT
   <213> Artificial Sequence
<220>
   <223> camelid VHH domain
<400> 4
   Val Gln Leu Leu Glu Ser Gly Gly Gly Leu Val Gln Ala Gly Gly Ser
   1               5                   10                  15
   Leu Thr Leu Ser Cys Glu Ala Ser Gly Leu Thr Phe Ser Glu Tyr Val
               20                  25                  30
   Met Gly Trp Phe Arg Gln Thr Pro Gly Lys Glu Arg Glu Phe Val Ala
           35                  40                  45
   Ala Val Ala Ala Met Asn Ser Arg Thr Tyr Tyr Thr Asp Ser Val Lys
       50                  55                  60
   Gly Arg Phe Thr Ile Ser Arg Asp Asn Ala Lys Asn Thr Val Phe Leu
   65                  70                  75                  80
   Gln Met Asn Ser Leu Lys Pro Glu Asp Thr Ala Val Tyr Tyr Cys Ala
                   85                  90                  95
   Val Gly Ala Tyr Gly Asp Tyr Ala Gly Pro Ser His Phe His Ser Trp
               100                 105                 110
   Gly Gln Gly Thr Gln Val Thr Val Ser
           115                 120
<210> 5
   <211> 8
   <212> PRT
   <213> Artificial Sequence
<220>
   <223> camelid VHH CDR1
<400> 5
   Gly Asn Ile Ser Asn Ile Leu Ser
   1               5
<210> 6
   <211> 7
   <212> PRT
   <213> Artificial Sequence
<220>
   <223> camelid VHH CDR2
<400> 6
   Ile Thr Asn Asp Gly Asn Thr
   1               5
<210> 7
   <211> 11
   <212> PRT
   <213> Artificial Sequence
<220>
   <223> camelid VHH CDR3
<400> 7
   Asn Ala Ala Arg Trp Thr Gln Pro Arg Pro Ser
   1               5                   10
<210> 8
   <211> 115
   <212> PRT
   <213> Artificial Sequence
<220>
   <223> camelid VHH domain
<400> 8
   Val Gln Leu Glu Glu Ser Gly Gly Gly Leu Val Glu Pro Gly Gly Ser
   1               5                   10                  15
   Leu Arg Leu Ser Cys Ala Ala Ser Gly Asn Ile Ser Asn Ile Leu Ser
               20                  25                  30
   Met Gly Trp Phe Arg Gln Ala Pro Gly Lys Arg Arg Glu Leu Val Ala
           35                  40                  45
   Thr Ile Thr Asn Asp Gly Asn Thr Glu Tyr Arg Asp Ser Val Lys Asp
       50                  55                  60
   Arg Phe Thr Ile Ser Arg Asp Asn Ala Glu Lys Thr Tyr Tyr Leu Gln
   65                  70                  75                  80
   Met Asp Arg Leu Lys Leu Glu Asp Thr Ala Val Tyr Tyr Cys Asn Ala
                   85                  90                  95
   Ala Arg Trp Thr Gln Pro Arg Pro Ser Trp Gly Gln Gly Thr Gln Val
               100                 105                 110
   Thr Val Ser
           115
<210> 9
   <211> 8
   <212> PRT
   <213> Artificial Sequence
<220>
   <223> camelid VHH CDR1
<400> 9
   Gly Arg Thr Asp Ser Leu Tyr Ala
   1               5
<210> 10
   <211> 8
   <212> PRT
   <213> Artificial Sequence
<220>
   <223> camelid VHH CDR2
<400> 10
   Ile Thr Trp Ile Gly Gly Ala Val
   1               5
<210> 11
   <211> 17
   <212> PRT
   <213> Artificial Sequence
<220>
   <223> camelid VHH CDR3
<400> 11
   Ala Ser Thr Phe Arg Arg Ile Trp Asp Ala Glu Glu Ser Ile Phe Asp
   1               5                   10                  15
   Thr
<210> 12
   <211> 122
   <212> PRT
   <213> Artificial Sequence
<220>
   <223> camelid VHH domain
<400> 12
   Val Gln Leu Leu Glu Ser Gly Gly Gly Leu Val Gln Ala Gly Asp Ser
   1               5                   10                  15
   Leu Arg Leu Ser Cys Ala Ala Ser Gly Arg Thr Asp Ser Leu Tyr Ala
               20                  25                  30
   Val Gly Trp Val Arg Gln Ala Pro Gly Lys Asp Arg Glu Phe Val Ala
           35                  40                  45
   Ala Ile Thr Trp Ile Gly Gly Ala Val Leu Tyr Ala Asp Ser Val Lys
       50                  55                  60
   Gly Arg Phe Thr Ile Ser Lys Lys Phe Gly Gly Asn Thr Val Tyr Leu
   65                  70                  75                  80
   Gln Met Asn Asn Leu Ala Pro Glu Asp Thr Ala Val Tyr Ser Cys Ala
                   85                  90                  95
   Ser Thr Phe Arg Arg Ile Trp Asp Ala Glu Glu Ser Ile Phe Asp Thr
               100                 105                 110
   Trp Gly Gln Gly Thr Gln Val Thr Val Ser
           115                 120
<210> 13
   <211> 8
   <212> PRT
   <213> Artificial Sequence
<220>
   <223> camelid VHH CDR1
<400> 13
   Gly Asn Ser Phe Ser Ile Ser Asn
   1               5
<210> 14
   <211> 7
   <212> PRT
   <213> Artificial Sequence
<220>
   <223> camelid VHH CDR2
<400> 14
   Ile Thr Ser Gly Gly Val Ala
   1               5
<210> 15
   <211> 12
   <212> PRT
   <213> Artificial Sequence
<220>
   <223> camelid VHH CDR3
<400> 15
   His Cys Glu Ser Gly Phe Pro Thr Leu Ile Ala Tyr
   1               5                   10
<210> 16
   <211> 116
   <212> PRT
   <213> Artificial Sequence
<220>
   <223> camelid VHH domain
<400> 16
   Val Gln Leu Leu Glu Ser Gly Gly Gly Leu Val Gln Phe Gly Gly Ser
   1               5                   10                  15
   Leu Arg Leu Ser Cys Ala Ala Ser Gly Asn Ser Phe Ser Ile Ser Asn
               20                  25                  30
   Met Gly Trp Tyr His Gln Ala Pro Gly Lys Glu Arg Glu Leu Val Ala
           35                  40                  45
   Ile Ile Thr Ser Gly Gly Val Ala Glu Tyr Ala Ala Ser Val Lys Gly
       50                  55                  60
   Arg Phe Thr Ile Ser Arg Asp Tyr Ala Lys Asn Thr Val Tyr Leu Gln
   65                  70                  75                  80
   Met Asn Ser Leu Gln Pro Glu Asp Thr Ala Val Tyr Tyr Cys His Cys
                   85                  90                  95
   Glu Ser Gly Phe Pro Thr Leu Ile Ala Tyr Trp Gly Gln Gly Thr Gln
               100                 105                 110
   Val Thr Val Ser
           115
<210> 17
   <211> 138
   <212> PRT
   <213> Artificial Sequence
<220>
   <223> single domain antibody
<400> 17
   Ser Gly Asp Ala Ser Asp Ser Gly Cys Val Gln Leu Leu Glu Ser Gly
   1               5                   10                  15
   Gly Gly Leu Val Gln Ala Gly Gly Ser Leu Thr Leu Ser Cys Glu Ala
               20                  25                  30
   Ser Gly Leu Thr Phe Ser Glu Tyr Val Met Gly Trp Phe Arg Gln Thr
           35                  40                  45
   Pro Gly Lys Glu Arg Glu Phe Val Ala Ala Val Ala Ala Met Asn Ser
       50                  55                  60
   Arg Thr Tyr Tyr Thr Asp Ser Val Lys Gly Arg Phe Thr Ile Ser Arg
   65                  70                  75                  80
   Asp Asn Ala Lys Asn Thr Val Phe Leu Gln Met Asn Ser Leu Lys Pro
                   85                  90                  95
   Glu Asp Thr Ala Val Tyr Tyr Cys Ala Val Gly Ala Tyr Gly Asp Tyr
               100                 105                 110
   Ala Gly Pro Ser His Phe His Ser Trp Gly Gln Gly Thr Gln Val Thr
           115                 120                 125
   Val Ser Cys Ser Gly Ser Thr Gly Glu Asn
       130                 135
<210> 18
   <211> 132
   <212> PRT
   <213> Artificial Sequence
<220>
   <223> single domain antibody
<400> 18
   Ser Gly Asp Ala Ser Asp Ser Gly Cys Val Gln Leu Glu Glu Ser Gly
   1               5                   10                  15
   Gly Gly Leu Val Glu Pro Gly Gly Ser Leu Arg Leu Ser Cys Ala Ala
               20                  25                  30
   Ser Gly Asn Ile Ser Asn Ile Leu Ser Met Gly Trp Phe Arg Gln Ala
           35                  40                  45
   Pro Gly Lys Arg Arg Glu Leu Val Ala Thr Ile Thr Asn Asp Gly Asn
       50                  55                  60
   Thr Glu Tyr Arg Asp Ser Val Lys Asp Arg Phe Thr Ile Ser Arg Asp
   65                  70                  75                  80
   Asn Ala Glu Lys Thr Tyr Tyr Leu Gln Met Asp Arg Leu Lys Leu Glu
                   85                  90                  95
   Asp Thr Ala Val Tyr Tyr Cys Asn Ala Ala Arg Trp Thr Gln Pro Arg
               100                 105                 110
   Pro Ser Trp Gly Gln Gly Thr Gln Val Thr Val Ser Cys Ser Gly Ser
           115                 120                 125
   Thr Gly Glu Asn
       130
<210> 19
   <211> 415
   <212> PRT
   <213> Artificial Sequence
<220>
   <223> single domain antibody with 3xFLAG-tag and GFP-derivative
<400> 19
   Ser Gly Asp Ala Ser Asp Ser Glu Val Gln Leu Leu Glu Ser Gly Gly
   1               5                   10                  15
   Gly Leu Val Gln Ala Gly Asp Ser Leu Arg Leu Ser Cys Ala Ala Ser
               20                  25                  30
   Gly Arg Thr Asp Ser Leu Tyr Ala Val Gly Trp Val Arg Gln Ala Pro
           35                  40                  45
   Gly Lys Asp Arg Glu Phe Val Ala Ala Ile Thr Trp Ile Gly Gly Ala
       50                  55                  60
   Val Leu Tyr Ala Asp Ser Val Lys Gly Arg Phe Thr Ile Ser Lys Lys
   65                  70                  75                  80
   Phe Gly Gly Asn Thr Val Tyr Leu Gln Met Asn Asn Leu Ala Pro Glu
                   85                  90                  95
   Asp Thr Ala Val Tyr Ser Cys Ala Ser Thr Phe Arg Arg Ile Trp Asp
               100                 105                 110
   Ala Glu Glu Ser Ile Phe Asp Thr Trp Gly Gln Gly Thr Gln Val Thr
           115                 120                 125
   Val Ser Ser Glu Pro Lys Thr Pro Lys Pro Gln Thr Ser Gly Ser Thr
       130                 135                 140
   Gly Glu Asn Leu Tyr Phe Gln Gly Asn Asp Tyr Lys Asp His Asp Gly
   145                 150                 155                 160
   Asp Tyr Lys Asp His Asp Ile Asp Tyr Lys Asp Asp Asp Asp Lys Thr
                   165                 170                 175
   Gly Thr Ser Lys Gly Glu Glu Leu Phe Thr Gly Thr Val Pro Ile Lys
               180                 185                 190
   Val Glu Leu Asp Gly Asp Val Asn Gly His Lys Phe Ser Val Arg Gly
           195                 200                 205
   Glu Gly Glu Gly Asp Ala Thr Glu Gly Lys Leu Thr Leu Lys Phe Ile
       210                 215                 220
   Cys Thr Thr Gly Lys Leu Pro Val Pro Trp Pro Thr Leu Val Thr Thr
   225                 230                 235                 240
   Leu Thr Tyr Gly Val Gln Cys Phe Ser Arg Tyr Pro Asp His Met Lys
                   245                 250                 255
   Arg His Asp Phe Phe Lys Ser Ala Met Pro Glu Gly Tyr Val Gln Glu
               260                 265                 270
   Arg Thr Ile Glu Phe Lys Asp Asp Gly Thr Tyr Lys Thr Arg Ala Glu
           275                 280                 285
   Val Lys Phe Glu Gly Asp Thr Leu Val Asn Arg Ile Glu Leu Lys Gly
       290                 295                 300
   Asn Asp Phe Lys Glu Asp Gly Asn Ile Leu Gly His Lys Leu Glu Tyr
   305                 310                 315                 320
   Asn His Asn Ser His Asn Val Arg Ile Glu Ala Asp Lys Gln Lys Asn
                   325                 330                 335
   Gly Ile Lys Ala Asn Phe Lys Ile Arg His Asn Val Glu Asp Gly Ser
               340                 345                 350
   Gln Gln Glu Ala Asp His Lys Gln Gln Asn Thr Pro Ile Gly Asp Gly
           355                 360                 365
   Pro Val Arg Leu Pro Asp Asn His Tyr Leu Ser Thr Gln Thr Thr Leu
       370                 375                 380
   Ser Lys Asp Pro Asn Glu Lys Arg Asp His Met Val Leu Lys Glu Phe
   385                 390                 395                 400
   Val Thr Ala Ala Gly Ile Thr Lys Gly Glu Asp Glu Arg Asp Lys
                   405                 410                 415
<210> 20
   <211> 133
   <212> PRT
   <213> Artificial Sequence
<220>
   <223> single domain antibody
<400> 20
   Ser Gly Asp Ala Ser Asp Ser Gly Cys Val Gln Leu Leu Glu Ser Gly
   1               5                   10                  15
   Gly Gly Leu Val Gln Phe Gly Gly Ser Leu Arg Leu Ser Cys Ala Ala
               20                  25                  30
   Ser Gly Asn Ser Phe Ser Ile Ser Asn Met Gly Trp Tyr His Gln Ala
           35                  40                  45
   Pro Gly Lys Glu Arg Glu Leu Val Ala Ile Ile Thr Ser Gly Gly Val
       50                  55                  60
   Ala Glu Tyr Ala Ala Ser Val Lys Gly Arg Phe Thr Ile Ser Arg Asp
   65                  70                  75                  80
   Tyr Ala Lys Asn Thr Val Tyr Leu Gln Met Asn Ser Leu Gln Pro Glu
                   85                  90                  95
   Asp Thr Ala Val Tyr Tyr Cys His Cys Glu Ser Gly Phe Pro Thr Leu
               100                 105                 110
   Ile Ala Tyr Trp Gly Gln Gly Thr Gln Val Thr Val Ser Cys Ser Gly
           115                 120                 125
   Ser Thr Gly Glu Asn
       130